# EUROPEAN PATENT SPECIFICATION

(11) **EP 4 608 553 B1**
(45) Date of publication and mention of the grant of the patent: **14.10.2026**
(21) Application number: 23809840.4
(22) Date of filing: 27.10.2023
(51) Int. Cl.: B01J 31/16, B01J 31/18, B01J 31/24, C07C 45/50, C07F 7/00, C07F 3/00, C07F 7/18, C07F 15/04, C07C 47/02

(54) **HETEROGENEOUS CATALYSTS**
HETEROGENE KATALYSATOREN
CATALYSEURS HÉTÉROGÈNES

(30) Priority: 18.11.2022 US 202263426436 P
(43) Date of publication of application: 03.09.2025
(73) Proprietor: DOW GLOBAL TECHNOLOGIES LLC, Midland, MI 48674 (US); MASSACHUSETTS INSTITUTE OF TECHNOLOGY, Cambridge, MA 02139 (US)
(72) Inventor: BEDARD, Anne-Catherine, Midland, Michigan 48674 (US); HOSSEININASAB, Valiallah, Cambridge, Massachusetts 02142 (US); DINCA, Mircea, Cambridge, Massachusetts 02139 (US); PAYNE, Michael Tyrel, Cambridge, Massachusetts 02139 (US); BOUR, James, Detroit, Michigan 48202 (US)
(74) Representative: Boult Wade Tennant LLP
(86) International application number: PCT/US2023/036096
(87) International publication number: WO 2024/107323

(56) References cited:
- US-A1- 2017 173 572
- MADHAN VINU ET AL: "Tetrazine-Based Metal-Organic Frameworks as Scaffolds for Post-Synthetic Modification by the Click Reaction", EUROPEAN JOURNAL OF INORGANIC CHEMISTRY, WILEY-VCH VERLAG , WENHEIM, DE, vol. 2020, no. 5, 22 January 2020 (2020-01-22), pages 461 - 466, XP072136485, ISSN: 1434-1948, DOI: 10.1002/EJIC.201901230
- LO SHENG-HAN ET AL: "Publisher Correction: Rapid desolvation-triggered domino lattice rearrangement in a metal-organic framework", NATURE CHEMISTRY, vol. 12, no. 1, 1 January 2020 (2020-01-01), London, pages 90 - 98, XP093127071, ISSN: 1755-4330, Retrieved from the Internet <URL:http://www.nature.com/articles/s41557-019-0404-9> [retrieved on 20240202], DOI: 10.1038/s41557-019-0404-9
- LI NA ET AL: "Functionalizing MOF with Redox-Active Tetrazine Moiety for Improving the Performance as Cathode of Li-O 2 Batteries", CCS CHEMISTRY, vol. 3, no. 3, 1 March 2021 (2021-03-01), pages 1297 - 1305, XP093127068, ISSN: 2096-5745, Retrieved from the Internet <URL:https://dx.doi.org/10.31635/ccschem.020.202000284> [retrieved on 20240202], DOI: 10.31635/ccschem.020.202000284
- SAMANTA PARTHA ET AL: "Heterogenized Molecular Rhodium Phosphine Catalyst within Metal-Organic Framework for Ethylene Hydroformylation", 16 September 2022 (2022-09-16), XP093127168, Retrieved from the Internet <URL:https://chemrxiv.org/engage/api-gateway/chemrxiv/assets/orp/resource/item/63244fdf0e3c6a824b1cfda8/original/heterogenized-molecular-rhodium-phosphine-catalyst-within-metal-organic-framework-for-ethylene-hydroformylation.pdf> [retrieved on 20240205], DOI: 10.26434/chemrxiv-2022-jcdpn
- PAYNE MICHAEL T ET AL: "Supporting Well-Defined Platinum Group Metals in Metal-Organic Frameworks for Heterogeneous Catalysis", 1 January 2023 (2023-01-01), XP093126882, Retrieved from the Internet <URL:https://dspace.mit.edu/bitstream/handle/1721.1/147541/payne-paynemic-phd-chemistry-2022-thesis.pdf?sequence=1&isAllowed=y> [retrieved on 20240202]

## Description

### Field of Disclosure

Embodiments of the present disclosure are directed towards catalysts and in particular heterogeneous catalysts.

### Background

Hydroformylation is a process in which olefins are converted to aldehydes in the presence of synthesis gas (carbon monoxide and hydrogen) and a catalyst under appropriate reaction conditions. The resulting aldehydes can then be further converted to any number of compounds, including alcohols, amines, carboxylic acids among other products. Hydroformylation can also be used in the synthesis of fine chemicals for use in pharmaceuticals, cosmetics and electronics, among other areas.

The catalysts used for hydroformylation have typically been homogeneous catalysts because of their ability to operate at relatively mild temperatures while exhibiting high activity and selectivity. However, many homogenous reactions are not commercially viable because of catalyst lifetime or recovery problems. For example, depending upon the size or functionality of the olefin involved, different types of hydroformylation purification processes can be used. When smaller olefins are used, such as propene and 1-butene, a vaporization process affords a straightforward separation of the organic products from the catalyst. However, higher or functional olefins, such as vinyl silanes, cannot be effectively separated with such technique due to their increased boiling point. In these cases, the distillation process often results in catalyst decomposition.

Attention, therefore, has been directed toward the attachment of homogeneous catalysts to solid supports in an attempt to combine the advantages of a heterogenous catalyst separation from the reaction products with the catalytic efficiency of a homogeneous system. Immobilization of the catalyst on a solid support, however, often results in a significant decrease in the selectivity and activity of the catalyst. So, despite the process advantages of heterogeneous catalysts, many reactions are still catalyzed using homogeneous catalysts. As a result, there is a need in the art to find ways of improving not only the selectivity and the activity of heterogeneous catalysts, but also allowing for heterogeneous catalysts to be recycled. US 2017/173572 A1 and Samanta Partha et al, "Heterogenized Molecular Rhodium Phosphine Catalyst within Metal-Organic Framework for Ethylene Hydroformylation", 2022-09-16, DOl: 10.26434/chemrxiv-2022-jcdpn disclose MOFs with phosphor-containing ligands coordinated to Group VIII metals as catalysts, i.a., for hydroformylation reactions.

### Summary

The present invention is disclosed in the appended claims.

The present disclosure addresses the above identified shortcomings by providing heterogeneous catalyst compositions that can provide the selectivity and activity comparable to that of their homogeneous counterpart while also being readily separated from the reaction mixture, providing the possibility for improved catalyst recyclability and lifetime.

The heterogenous catalyst composition of the present disclosure is formed using a crystalline porous metal-organic framework (MOF) which reacts in an inverse-electron-demand Diels-Alder (IEDDA) reaction with a suitable dienophile bearing a phosphorous ligand to produce a heterogeneous catalyst precursor. The heterogeneous catalyst precursor then reacts with a Group VIII transition metal catalyst precursor compound to form the heterogeneous catalyst composition that can be used for the hydroformylation of olefins, among other reactions. For example, an aldehyde can be produced in the hydroformylation of an olefin in the presence of synthesis gas and the heterogeneous catalyst composition of the present disclosure. Alternatively, the MOF reacts in an IEDDA reaction with a pre-formed metal phosphine complex to directly produce the heterogeneous catalyst composition.

For the various embodiments, the crystalline porous MOF includes a plurality of non-catalytic metal ions and a plurality of linkers, where the plurality of non-catalytic metal ions coordinates with the plurality of linkers to form the crystalline porous MOF. The plurality of linkers are formed from a compound of Formula I: 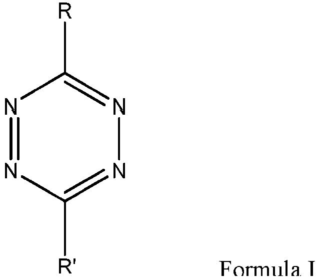 where each of R and R' is independently selected from:

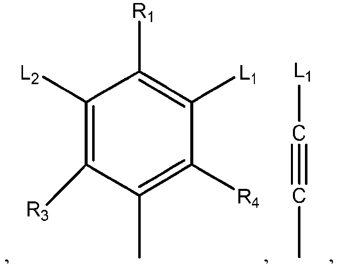

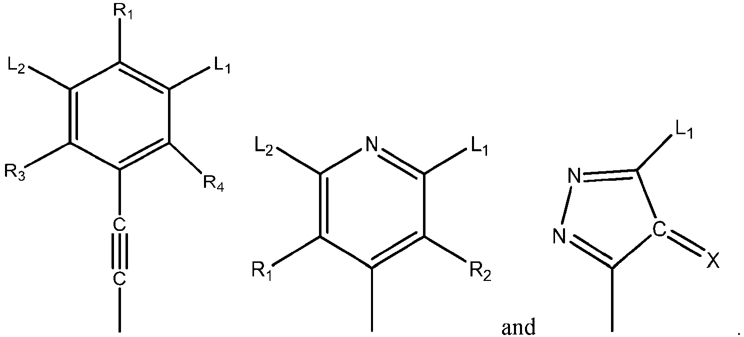

Each of R₁ and R₂ are independently selected from -H, C₁ to C₃ alkyl, -F, - Cl, -Br, -I, or -CF₃; each of R₃ and R₄ are independently selected from -H or C₁ to C₃ alkyl, where R₃ and R₄ can form a bridge structure when both are alkyl groups; X is selected from O and S; L₁ and L₂ are each selected from the group: 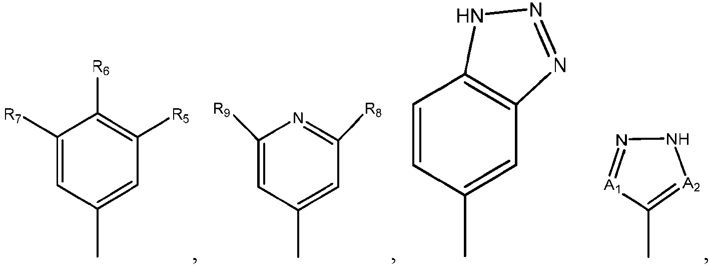 -COOH, -OH and - NH₂. Each of R₅, R₆ and R₇ are independently selected from -H -OR₁₀, -COOR₁₁, -NR₁₂, -F, - Cl, -Br, -I, or -CF₃, wherein R₁₀, R₁₁ and R₁₂ are each independently selected from -H or C₁ to C₃ alkyl; each R₈ and R₉ are independently selected from -H, -OH, -COOH, or -NH₂; and A₁ and A₂ are either C or N.

In one embodiment, each of R and R' of the crystalline porous MOF is: 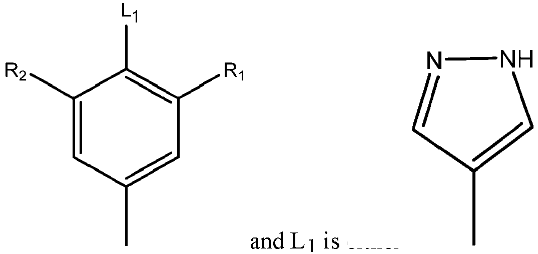 For the various embodiments, R₁ is -H, -F or -CF₃, and R₂ is -H. In an additional embodiment, L₁ is 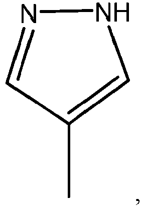 where R₁ is -F or -CF₃ and R₂ is -H.

In another embodiment, each of R and R' is: 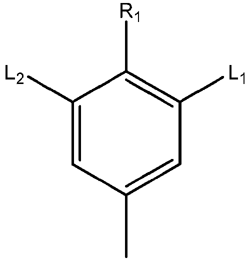 and L₁ and L₂ are 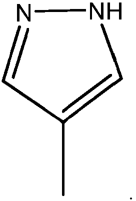 For the various embodiments, R₁ can be H.

The plurality of non-catalytic metal ions for the crystalline porous MOF can be selected from the group consisting of nickel (Ni), magnesium (Mg), copper (Cu), cobalt (Co), zirconium (Zr), iron (Fe), zinc (Zn), vanadium (V), aluminum (Al) and combinations thereof. The non-catalytic metal ions are derived from metal ion salts, as discussed herein.

The heterogenous catalyst precursor is formed from an IEDDA reaction product of the crystalline porous MOF and a dienophile that contains a phosphorous ligand. In one embodiment, the phosphorous ligand can be of Formula II: 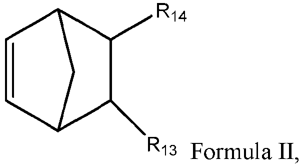 where the reaction forms the heterogeneous catalyst precursor of Formula III:

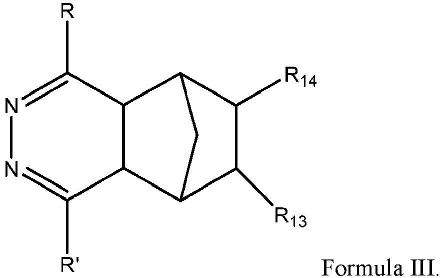

In an alternative embodiment, the phosphorous ligand can be of Formula IV: 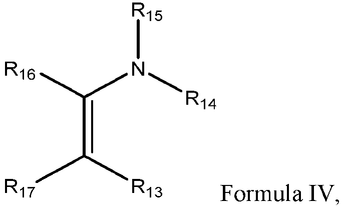 where the reaction forms the heterogeneous catalyst precursor of Formula V: 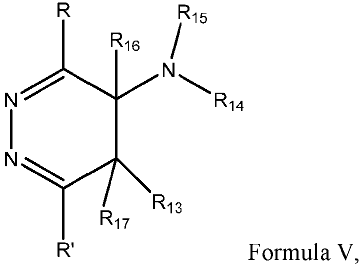 or

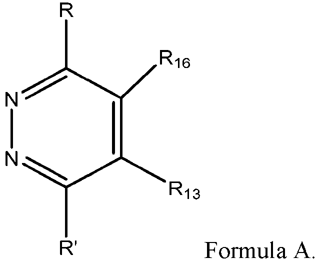

In an additional embodiment, the phosphorous ligand can be of Formula VI: 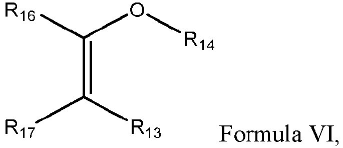 where the reaction forms the heterogeneous catalyst precursor of Formula VII: 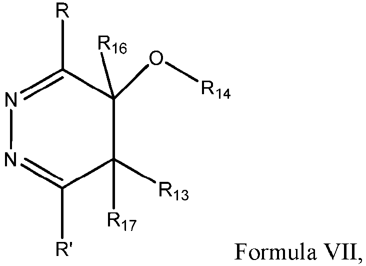 or

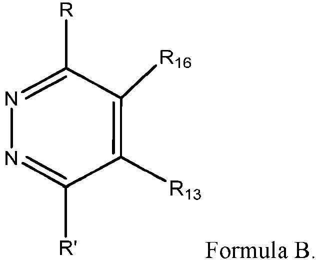

In an additional embodiment, the phosphorous ligand can be of Formula VIII or Formula C: 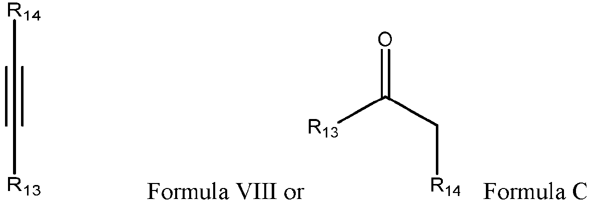 and an amine catalyst, where the reaction forms the heterogeneous catalyst precursor of Formula IX:

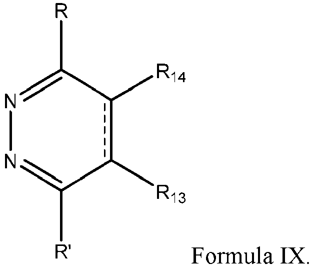

With respect to the use of Formula C, an enamine can be generated *in situ* by condensation of the ketone of Formula C and an appropriate amine catalyst, such as pyrrolidine according to the following reaction: 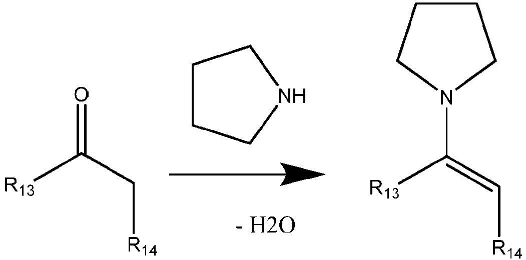 where the IEDDA reaction produces the catalyst precursor of the present disclosure, but eliminates the amine to produce the structure of Formula IX as follows:

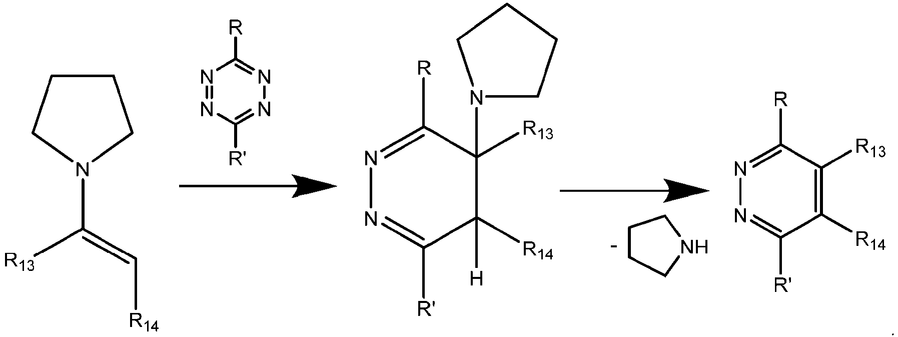

In an additional embodiment, the phosphorous ligand can be of Formula D:

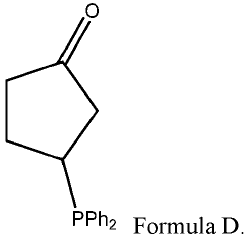

For the above formula II through IX, R₁₆ and R₁₇ are each independently selected from -H and C₁ to C₃ alkyl, and R₁₃ - R₁₅ are each independently selected from - H, C₁ to C₃₀ alkyl and at least one of: 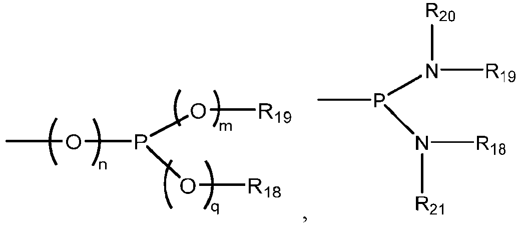 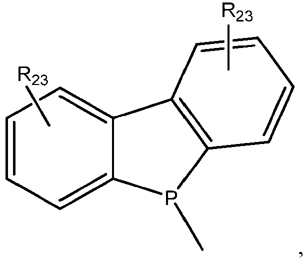 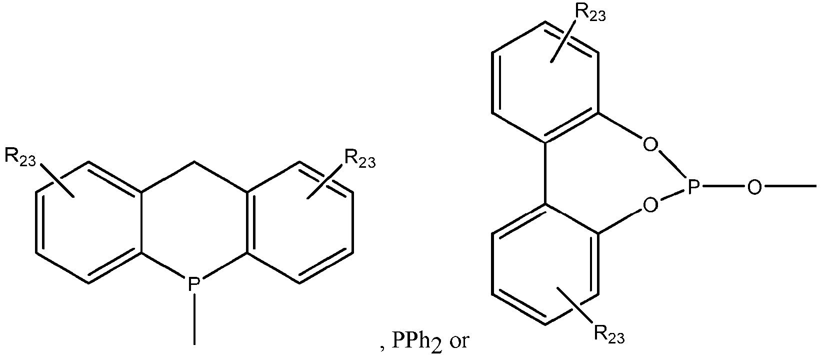 , where n, m, and q are either 0 or 1 and where (n,m,q) is either (1,0,0), (1,1,0), (0,0,1), (0,1,1) or (1,1,1). Each of R₁₈ through R₂₁ are selected from C₅-C₁₀ aryl, C₁-C₄ alkyl or - N(R₂₂)₂, where R₂₂ is selected from -H or C₁ to C₃ alkyl. Each of R₂₃ is selected from C₅-C₁₀ aryl, C₁-C₄ alkyl, -OR₂₂ or -N(R₂₂)₂, where R₂₂ is selected from -H or C₁ to C₃ alkyl.

The heterogeneous catalyst precursor can then react with a Group VIII transition metal catalyst precursor compound to form the heterogeneous catalyst composition that can be used for the hydroformylation of olefins. For the various embodiments, the Group VIII transition metal catalyst precursor compound is of Formula X:

M_{w}(L¹)ₓ(L²)_{y}(L³)_{z} Formula X

where the M is selected from the group consisting of rhodium (Rh), cobalt (Co), iridium (Ir), ruthenium (Ru), iron (Fe), nickel (Ni), palladium (Pd), platinum (Pt), and osmium (Os). For the various embodiments, L¹, L² and L³ are each independently selected from the group consisting of hydrogen, carbonyl (CO), cyclooctadiene, norbornene, chlorine, oxygen, boron, fluoride, bromide, iodide, nitrate, acetate, octanoate, 2-ethylhexanoate, triphenylphosphine (TPP), and acetylacetonate (AcAc). For Formula X, w is an integer from 1 to 6, and x, y and z are each independently an integer from 0 to 5 wherein the sum of x, y, and z is at least 1.0. For the various embodiments, the transition metal catalyst of Formula X is selected from the group consisting of Rh(O₂C₅H₇)(CO)₂, Rh₂O₃, Rh₄(CO)₁₂, Rh₆(CO)₁₆, Rh(NO₃)₃, bis(norbornadiene)rhodium(I) tetrafluoroborate and bis(1,5-cyclooctadiene)rhodium(I) tetrafluoroborate. Precursors can also include tris(triphenylphosphine)rhodium carbonyl hydride and acetylacetonatocarbonyltriphenylphosphinerhodium(I).

For the various embodiments, the present disclosure further provides for a method of forming the heterogeneous catalyst composition that includes reacting the compound of Formula I with a metal acetate to form the crystalline porous MOF; reacting a phosphorous ligand of any one of Formula II, Formula IV, Formula VI or Formula VIII with the crystalline porous MOF in an inverse electron-demand Diels-Alder reaction to form the heterogeneous catalyst precursor of Formula III, Formula V, Formula VII or Formula IX, respectively; and reacting the Group VIII transition metal catalyst precursor compound with the heterogeneous catalyst precursor to form the heterogeneous catalyst composition of the present disclosure.

For the various embodiments, producing an aldehyde according to the present disclosure is accomplished with a method that includes providing a reaction mixture of an C3 to C12 olefin, synthesis gas and the heterogeneous catalyst composition provided herein; and reacting the C3 to C12 olefin with synthesis gas in the presence of the heterogeneous catalyst composition in a hydroformylation process to produce the aldehyde. For the various embodiments, the hydroformylation process is conducted in a fixed bed process. For the various embodiments, the present disclosure further includes separating the heterogeneous catalyst composition from the reaction mixture after producing the aldehyde using one of a filtration process, a membrane separation process or a centrifuge separation process.

The above summary of the present disclosure is not intended to describe each disclosed embodiment or every implementation of the present disclosure. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### Detailed Description

All references to the Periodic Table of the Elements and the various groups therein are to the version published in the CRC Handbook of Chemistry and Physics, 72nd Ed. (1991-1992) CRC Press, at page 1-11.

Unless stated to the contrary, or implicit from the context, all parts and percentages are based on weight and all test methods are current as of the filing date of this application.

As used herein, "a," "an," "the," "at least one," and "one or more" are used interchangeably. The terms "comprises," "includes," and variations thereof do not have a limiting meaning where these terms appear in the description and claims. Thus, for example, an aqueous composition that includes particles of "a" hydrophobic polymer can be interpreted to mean that the composition includes particles of "one or more" hydrophobic polymers.

As used herein, the term "ppm" means parts per million by weight.

As used herein, "Ph" is phenyl or substituted phenyl.

As used herein, the term "aryl" as used herein is a group containing any carbon-based aromatic group including, but not limited to, benzene, naphthalene, phenyl, biphenyl, anthracene, and the like. The aryl group may be substituted or unsubstituted. Aryl groups are aryl, heteroaryl as described herein. It may be substituted with one or more groups including but not limited to halide, hydroxy, ketone, azide, nitro, silyl, sulfo-oxo or thiol. The term "biaryl" is a specific type of aryl group and is included in the definition of "aryl". Furthermore, an aryl group can be a single ring structure, or a plurality of rings that are either fused ring structures or linked via one or more bridging groups, such as carbon-carbon bonds. Includes structure. For example, a biaryl is bonded together via a fused ring structure, as in naphthalene, or via two or more carbon-carbon bonds, as in biphenyl. Refers to an aryl group.

As used herein, the term "alkyl" is a branched (when possible) or unbranched saturated hydrocarbon group of the number of carbon atoms specified. Examples of alkyl groups include, but are not limited to, methyl, ethyl, n-propyl, Isopropyl, n-butyl, isobutyl, s-butyl, t-butyl, n-pentyl, isopentyl, s-pentyl, neopentyl, hexyl, heptyl, octyl, nonyl, decyl, dodecyl, tetradecyl, hexadecyl, eicosyl, tetracosyl, *etc.* The alkyl group may be substituted or unsubstituted.

For purposes of this disclosure, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds

As used herein, the term "substituted" is contemplated to include all permissible substituents of organic compounds unless otherwise indicated. In a broad aspect, the permissible substituents include acyclic and cyclic, branched and unbranched, carbocyclic and heterocyclic, aromatic and nonaromatic substituents of organic compounds. Illustrative substituents include, for example, alkyl, alkyloxy, aryl, aryloxy, hydroxy, hydroxyalkyl, amino, aminoalkyl, halogen and the like in which the number of carbons can range from 1 to about 20 or more, alternatively from 1 to about 12. The permissible substituents can be one or more and the same or different for appropriate organic compounds.

Also herein, the recitations of numerical ranges by endpoints include all numbers subsumed in that range *(e.g.,* 1 to 5 includes 1, 1.5, 2, 2.75, 3, 3.80, 4, 5, etc.). For the purposes of the present disclosure, it is to be understood, consistent with what one of ordinary skill in the art would understand, that a numerical range is intended to include and support all possible subranges that are included in that range. For example, the range from 1 to 100 is intended to convey from 1.01 to 100, from 1 to 99.99, from 1.01 to 99.99, from 40 to 60, from 1 to 55, etc.

As used herein, the term "hydroformylation" is contemplated to include, but not limited to, all permissible asymmetric and non-asymmetric hydroformylation processes that involve converting one or more substituted or unsubstituted olefinic compounds or a reaction mixture comprising one or more substituted or unsubstituted olefinic compounds to one or more substituted or unsubstituted aldehydes or a reaction mixture comprising one or more substituted or unsubstituted aldehydes.

Embodiments of the present disclosure provide for a heterogenous catalyst composition formed from a phosphorus ligand, covalently bound to a crystalline porous metal-organic framework (MOF), where the phosphorus ligand binds a transition metal catalyst precursor compound to provide the heterogenous catalyst composition of the present disclosure. As discussed herein, the phosphorous ligand of the present disclosure provides for design control (e.g., control of steric and electronic properties) that can lead to desirable selectivity and activity from the heterogeneous catalyst composition of the present disclosure. The crystalline porous MOF of the present disclosure is composed of inorganic clusters (e.g., non-catalytic metal ions) connected by linkers bearing a tetrazine group, as discussed herein. The crystalline porous MOF provides a solid-state crystalline and porous network on which to modulate and tune the design control of the phosphorous ligand. The crystalline porous MOF also provides the solid phase that allows for the heterogenous catalyst composition of the present disclosure to be readily separated from the reaction mixture (e.g., olefins, aldehydes and synthesis gas in a hydroformylation reaction), thus improving catalyst recyclability and lifetime.

For the various embodiments, the present disclosure utilizes what is referred to herein as a tetrazine based "click-grafting" reaction to covalently link the phosphorus ligand of the present disclosure to the crystalline porous MOF to form what is referred to herein as the heterogenous catalyst precursor. Until this disclosure, one issue with trying to covalently bond a phosphorous ligand to a conventional MOF is that phosphorus ligands are generally prone to decompose in the harsh synthesis conditions used in forming the MOFs (often by oxidation or hydrolysis). The phosphorus ligands can also compete with the linker for coordination to the non-catalytic metal clusters in the MOF, which is not desirable.

To address the above noted issues, the present disclosure uses the tetrazine based "click-grafting" reaction to covalently bond the phosphorus ligand to a tetrazine moiety that is present on the crystalline porous MOF of the present disclosure. The advantage of the tetrazine-based click-grafting reaction are that it is rapid, traceless, and forms strong covalent bonds, which allow the phosphorus ligand to be firmly anchored to the crystalline porous MOF without disrupting its porous crystalline structure. Another advantage of the click grafting procedure is that the phosphorous moieties do not interfere or react with the components of the click grafting process

The tetrazine-based click-grafting reaction of the present disclosure proceeds via a 4+2 cycloaddition reaction (e.g., an IEDDA reaction) that rapidly and selectively bonds the phosphorous ligand to the tetrazine moiety in the crystalline porous MOF. To facilitate this reaction, the phosphorous ligand of the present disclosure includes what is referred to herein as a "click-reaction partner," which can be a strained and/or electron rich dienophile (*i.e.,* a compound having a multiple bond such as an alkene, an alkyne, an allyl, an enamines, or enol ether) that reacts with the tetrazine moiety in the crystalline porous MOF. Such a reaction occurs under mild conditions, where only a nitrogen (N₂) byproduct is produced in forming the heterogenous catalyst precursor of the present disclosure. The phosphorus ligand of the present disclosure further includes a reaction moiety that reacts with a Group VIII transition metal catalyst precursor compound, as provided herein, to form the heterogenous catalyst of the present disclosure.

As provided herein, the heterogenous catalyst of the present disclosure can have the activity and selectivity of their homogeneous counterpart. The heterogeneous catalyst composition of the present disclosure may be useful in a broad classes of reactions such as hydroformylation, carbonylation of alcohols, cross-coupling and asymmetric hydrogenation, and olefin (e.g., ethylene) oligomerization, among other reactions provided herein.

The present disclosure provides for heterogeneous catalyst compositions that can provide the selectivity and activity comparable to that of their homogeneous counterpart while also being readily separated from the reaction mixture, providing the possibility for improved catalyst recyclability and lifetime. The heterogenous catalyst of the present disclosure is formed from using the crystalline porous MOF which reacts in an IEDDA reaction with a suitable dienophile containing the phosphorous ligand to produce the heterogeneous catalyst precursor. The heterogeneous catalyst precursor then reacts with the Group VIII transition metal catalyst precursor compound to form the heterogeneous catalyst composition that can be used for the hydroformylation of olefins. Alternatively, the MOF reacts in an IEDDA reaction with a pre-formed metal phosphine complex to directly produce the heterogeneous catalyst composition. The above components of the present disclosure are disclosed as follows.

### Crystalline Porous MOF

The crystalline porous metal-organic framework (MOF) of the present disclosure can be a one-dimensional porous structure, a two-dimensional porous structure, or a three-dimensional porous structure composed of two major components: (a) linkers (sometimes referred to as "struts" or "ligands") that coordinate to (b) a plurality of non-catalytic metal ions (sometimes referred to as "clusters" or "nodes" or "secondary building units (SBUs)"). For the various embodiments, the crystalline porous MOF includes the plurality of non-catalytic metal ions and the plurality of linkers, where the plurality of non-catalytic metal ions coordinate with the plurality of linkers to form the crystalline porous MOF. The crystalline porous MOFs possess highly ordered structures with significantly high surface areas. Alternatively, the crystalline porous MOF of the present disclosure is highly porous. The crystalline porous MOF of the present disclosure can take the form of various particles *(e.g.,* flakes, rods, needles, *etc.).*

The crystalline porous MOF described herein comprise the plurality of non-catalytic metal ions forming the nodes or clusters, which may be formed with at least one metal selected from the group consisting of Sc, Ti, V, Cr, Mn, Fe, Co, Ni, Cu, Y, Zr, Nb, Mo, Tc, Ru, Rh, Pd, Ag, Hf, Ta, W, Re, Os, Ir, Pt, Au, Rf, Db, Sg, Bh, Hs, Mt, Ds, Rg, Cn, Al, In, Ga, Sn, Bi, Pb, Tl, Zn, Cd, Hg Be, Mg, Ca, Sr, Ba, and Ra. Alternatively, the plurality of non-catalytic metal ions for the crystalline porous MOF can be selected from the group consisting of nickel (Ni), magnesium (Mg), copper (Cu), cobalt (Co), zirconium (Zr), iron (Fe), zinc (Zn), vanadium (V), aluminum (Al) and combinations thereof. In an alternative embodiment, the non-catalytic metal is Ni, Mg and/or Zr. For the various embodiments, the non-catalytic metal ion may have any number of its possible oxidation states (*e.g.,* M⁺¹, M⁺², M⁺³). The non-catalytic metals provided herein are not relied upon to be catalytically active as they do not have a directly bonded phosphorous ligand.

For the various embodiments, the choice of the non-catalytic metal ion and the linker arm help dictate the structure and properties of the crystalline porous MOF. For example, the non-catalytic metal ion's coordination preference influences the size and shape of the pores of the crystalline porous MOF by dictating how many linkers can bind to the non-catalytic metal cluster or nodes and in which orientation. For the various embodiments, the linkers of the present disclosure can be multidentate, having at least two donor atoms *(i.e.,* bidentate atoms such as -N, -O, and/or -S) and being neutral or anionic. The structure of the crystalline porous MOF can also be affected by the shape, length, and functional groups present in the linker arm.

For the various embodiments, the plurality of linkers are formed from a compound of Formula I: 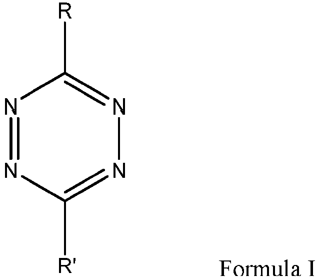 where each of R and R' is independently selected from:

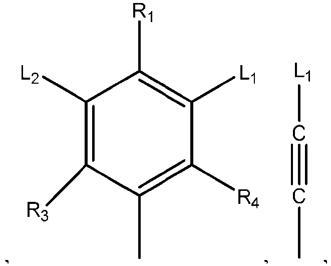

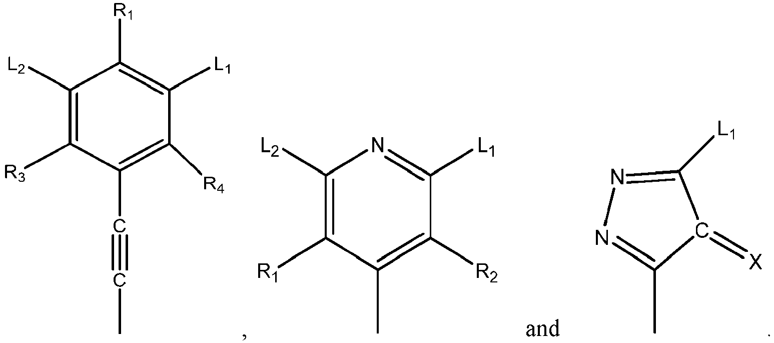

Preferably, R and R' are the same moiety. Alternatively, R and R' can be different moieties. For the various embodiments, the selection of the moiety for R and R' can be based on a desired length and geometry of the linker, where the length and geometry of the linker can allow for the design and tailoring of the porosity and dimensional structure of the resulting crystalline porous MOF.

For the various embodiments, each of R₁ and R₂ are independently selected from -H, C₁ to C₃ alkyl, -F, -Cl, -Br, -I, or -CF₃; each of R₃ and R₄ are independently selected from -H or C₁ to C₃ alkyl, where R₃ and R₄ can form a bridge structure when both are alkyl groups; Xis selected from O and S; L₁ and L₂ are each selected from the group: 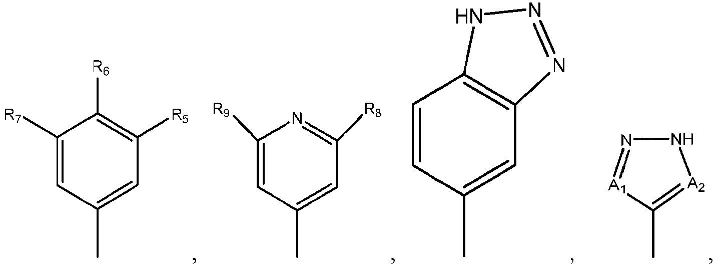 -COOH, -OH and - NH₂.
Each of R₅, R₆ and R₇ are independently selected from -H -OR₁₀, -COOR₁₁, -NR₁₂, -F, - Cl, -Br, -I, or --CF₃, where R₁₀, R₁₁ and R₁₂ are each independently selected from -H or C₁ to C₃ alkyl; each R₈ and R₉ are independently selected from -H, -OH, -COOH, or -NH₂; and A₁ and A₂ are either C or N.

In one embodiment, each of R and R' of the crystalline porous MOF is: 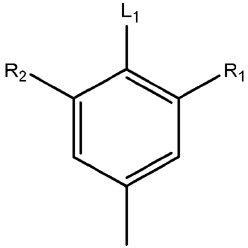 and L₁ is 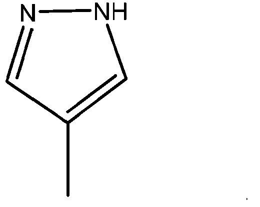 For the various embodiments, R₁ is -H, -F or -CF₃, and R₂ is -H. In an additional embodiment, L₁ is 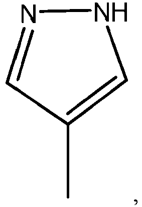 where R₁ is -F or -CF₃ and R₂ is -H.

In another embodiment, each of R and R' is: 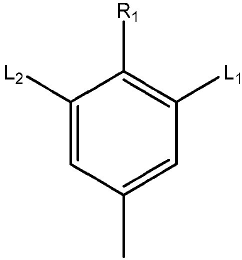 and L₁ and L₂ are 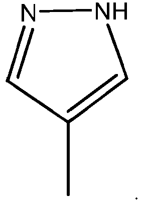 For the various embodiments, R₁ can be H.

Preparation of linkers are readily available to one of ordinary skill in the art. Examples of producing the linkers are provided in the Examples section herein. Typically, the linkers formed from a compound of Formula I are produced by oxidation of a dihydrotetrazine precursor. Examples of oxidizing agents for conversion of the dihydrotetrazine precursor to the tetrazine linker include isoamyl nitrite, hydrogen peroxide, FeCl₃, Cl₂, Br₂, NaNO₂/H⁺, 2,3-Dichloro-5,6-dicyano-1,4-benzoquinone (DDQ), N-bromosuccinimide (NBS), oxygen, among others. Typically, the dihydrotetrazine precursor is produced by Pinner synthesis involving the condensation of two equivalents of an aryl nitrile with hydrazine hydrate. The Pinner synthesis can be catalyzed by a sulfur catalyst or a Lewis acid. Examples of sulfur catalysts include S₈ and N-acetylcysteine. Examples of Lewis acid catalysts include metal salts of nickel and zinc. Alternatively, the dihydrotetrazine precursor to the linkers formed from a compound of Formula I are produced by reaction of imidoesters with hydrazine, or by treatment of acylhydrazides with PCl₅ and subsequent condensation with tetrazine, or by cyclization of a thiocarbohydrazide with a carboxylic acid or trithiocarbonate. With respect to their geometric shape, the crystalline porous MOF may be in the form of polyhedral crystals, cubic, hexagonal or octahedral in shape. For the various embodiments, each linker of the crystalline porous MOF can connect to two or more of the nodes of the plurality of non-catalytic metal ions. Such a configuration allows for each node of the plurality of non-catalytic metal ions may be coordinated in an octahedral geometry with, for example, twelve linkers. Alternatively, the configuration can allow for each node of the plurality of non-catalytic metal ions to be coordinated in a cubic or hexagonal geometry.

For the various embodiments, the crystalline porous MOF of the present disclosure can have a Brunauer-Emmet-Teller (BET) surface area of 500 to 5000 m²/g. Alternatively, the crystalline porous MOF of the present disclosure can have a BET surface area of 1000 to 4000 m²/g. BET surface area is measured according to S. Brunauer, P. H. Emmett, E. Teller, J. Am. Chem. Soc. 1938, 60, 309-319, incorporated herein by reference, using nitrogen gas adsorption analysis. Pore diameters for the crystalline porous MOF of the present disclosure can range from 0.1 nanometers (nm) to 5 nm, where there can be a mixture of pore diameters. Pore diameters can also range from 0.9 nm to 3 nm. For some embodiments, pore size distribution (PSD) is determined from nitrogen gas adsorption data using the Barrett, Joyner, and Halenda (BJH) method according to E. P. Barrett, L. G. Joyner, P. P Halenda, J. Am. Chem. Soc. 1951, 73, 1, 373-380, incorporated herein by reference. Alternatively, PSD is determined from nitrogen gas adsorption data using non-linear density functional theory (NLDFT) models according to N.A. Seaton, J.P.R.B. Walton, N. Quirke, Carbon, 1989, 27, 6, 853-861, incorporated herein by reference.

For the various embodiments, a single type of linker arm is used in forming the crystalline porous MOF of the present disclosure to provide the crystalline porous MOF with an undistorted geometry. In alterative embodiment, a first type of linker arm and a second type of linker arm *(e.g.,* the second type of linker arm is longer than the first linker arm) are used so that the crystalline porous MOF has a distorted geometry. Additional types of linkers might also be used, as desired, to provide additional distorted geometries for the crystalline porous MOF.

The crystalline porous MOF can be formed according to a number of different processes. Examples of such processes include, but are not limited to solvothermal methods, mechanochemical methods, electrochemistry methods, assisted synthesis methods *(i.e.,* by ultrasound or microwave), and subcritical water methods. For example, the crystalline porous MOF can be produced by a solvothermal method. Typically, a solvothermal synthesis comprises the reaction of one or more metal salts and one or more linkers, as provided herein, in the presence of organic solvents or mixtures, alternatively involving formamides, alcohols, or pyrrolidones. Parameters in the solvothermal synthesis include, but are not limited to, temperature, concentration of reactants (which can be varied over a wide range) and pH of the reaction solution. The method can include mixing a source of the plurality of non-catalytic metal ions, the linkers, an organic solvent, and water to produce a reaction mixture. The reaction mixture can be allowed to react for the time and temperature provided herein, where the reaction can occur under an atmosphere of room air and at atmospheric pressure. It is possible, however, that depending on the non-catalytic metal ion and linker arm, synthesis of the crystalline porous MOF may require use of an inert atmosphere like nitrogen gas (N₂).

Examples of the metal ion salts of the metals provided herein include, but are not limited to, nitrates, chlorates, sulfates, phosphates, molybdates, chromates, arsenites, acetates, bromides, chlorides, fluorides, silicates, iodides, methacrylates, among other, including their hydrated salt. Specific examples of such metal ion salts includes, but are not limited to, Ni(OAc)₂,Ni(NO₃)₂, ZrCl₄, ZrOCl₂, ZnCl₂, Mg(NO₃)₂, FeCl₂ and FeCl₃.

For the various embodiments, when forming the crystalline porous MOF the reaction mixture can contain a molar excess of the non-catalytic metal ion as compared to the linker arm. In one embodiment, a molar ratio of the non-catalytic metal ion to the linker arm in the mixture can be in a range of 1:1 to 2: 1, alternatively 1.1:1 to 1.5:1, or about 1.2:1 to 1.3:1. In one embodiment, non-catalytic metal ion is present in the reaction mixture at a concentration in a range of 0.05 to 0.5 M, alternatively 0.08 to 0.3 M, alternatively about 0.1 M.

In one embodiment, the organic solvent may be one or more solvents selected from ethers (e.g. diethyl ether, tetrahydrofuran, 1,4-dioxane, tetrahydropyran, t-butyl methyl ether, cyclopentyl methyl ether, di-iso-propyl ether), glycol ethers *(e.g., 1,2-*dimethoxyethane, diglyme, triglyme), alcohols (e.g., methanol, ethanol, trifluoroethanol, n-propanol, i-propanol, n-butanol, i-butanol, t-butanol, n-pentanol, i-pentanol, 2-methyl-2-butanol, 2-trifluoromethyl-2-propanol, 2,3-dimethyl-2-butanol, 3-pentanol, 3-methyl-3-pentanol, 2-methyl-3-pentanol, 2-methyl-2-pentanol, 2,3-dimethyl-3-pentanol, 3-ethyl-3-pentanol, 2-methyl-2-hexanol, 3-hexanol, cyclopropylmethanol, cyclopropanol, cyclobutanol, cyclopentanol, cyclohexanol), aromatic solvents (e.g., benzene, o-xylene, m-xylene, p-xylene, mixtures of xylenes, toluene, mesitylene, anisole, 1,2-dimethoxybenzene, α,α,α-trifluoromethylbenzene, fluorobenzene), chlorinated solvents (e.g., chlorobenzene, dichloromethane, 1,2-dichloroethane, 1,1-dichloroethane, chloroform), ester solvents (e.g., ethyl acetate, propyl acetate), amide solvents (e.g., dimethylformamide, diethylformamide, dimethylacetamide, N-methyl-2-pyrrolidone), urea solvents, ketones (e.g. acetone, butanone), acetonitrile, propionitrile, butyronitrile, benzonitrile, dimethyl sulfoxide, ethylene carbonate, propylene carbonate, 1,3-dimethyl-3,4,5,6-tetrahydro-2(1H)-pyrimidinone, and mixtures thereof. The solvent may be selected from non-polar solvents (e.g., hexane, benzene, toluene, diethyl ether, chloroform, 1,4-dioxane), polar aprotic solvents (e.g., ethyl acetate, tetrahydrofuran, dichloromethane, acetone, acetonitrile, dimethylformamide, dimethyl sulfoxide) and polar protic solvents (e.g., acetic acid, n-butanol, isopropanol, n-propanol, ethanol, methanol, formic acid, water) and mixtures thereof. Alternatively, the solvent is dimethylformamide (DMF) and water.

After the mixing, the reaction mixture is heated to produce a precipitate. The reaction mixture is heated at a temperature in a range of 100 to 180 °C, alternatively 120 to 170 °C, alternatively 130 to 160 °C or alternatively 145 to 155 °C for a time in a range of 1 hour to 5 days, alternatively 1 hour to 3 days (72 hours), alternatively about 24 hours to 48 hours. In one embodiment, the reaction mixture may be heated in a microwave oven or in an autoclave (or other enclosed vessel) at an elevated pressure. In one embodiment, the reaction mixture may be continually mixed or agitated during the heating. Alternatively, the reaction mixture may not be disturbed during the heating.

For the various embodiments, the use of a modulator in the reaction mixture is also possible. As used herein, a modulator is used to control the crystal growth in the crystalline porous MOF of the present disclosure. For the various embodiments, the modulator is often a monotopic analog to the linker arm (e.g., benzoic acid for a carboxylate MOF) and is often acidic, such that the modulator either competes with the linker arm for non-catalytic metal ion coordination, or the modulator lowers the pH and thus concentration of deprotonated linker arm. Basic modulators can also be used to promote the crystalline porous MOF formation by deprotonating highly basic linkers. Examples of the modulator includes, but is not limited to, benzoic acid, acetic acid, HCl, pyrazole, amines and pyridine.

The heating produces a precipitate comprising crystals of the crystalline porous MOF. Preferably, after the step of heating the reaction mixture, the reaction mixture is allowed to cool to room temperature. The crystalline porous MOF may be isolated and purified by methods known to those of ordinary skill in the art, such a filtration, decantation, aqueous work-up, extraction with organic solvents, on normal phase or reversed phase. The crystalline porous MOF may be rinsed with an organic solvent such as those previously mentioned, and then activated. In one embodiment, the crystalline porous MOF is activated at a temperature in a range of 40 to 150 °C under a high vacuum 0.00001 to 1 Pascal (Pa) for 8 to 72 hours.

### Heterogenous Catalyst Precursor

The heterogenous catalyst precursor of the present disclosure is formed as the reaction product of the crystalline porous MOF and a phosphorous ligand using an inverse electron-demand Diels-Alder (IEDDA) reaction. The reaction is referred to herein as a tetrazine based click-grafting reaction that covalently links the phosphorus ligand, as provided herein, to the crystalline porous MOF to form the heterogenous catalyst precursor of the present disclosure. The IEDDA reaction is a cycloaddition reaction that forms two new chemical bonds from the reaction between an electron-rich dienophile and an electron-poor diene. The IEDDA reaction occurs extremely rapidly (e.g., second order reaction kinetics between 800 M⁻¹s⁻¹ and 30000 M⁻¹s⁻¹). In the present disclosure, the IEDDA reaction proceeds between a strained and/or electron-rich alkene and a tetrazine moiety located on the linker arm of the present disclosure.

As is known in the art, the IEDDA reaction is very rapid yet proceeds under very mild conditions, producing nitrogen gas (N₂) as the sole reaction by-product. Reaction temperatures for the IEDDA reaction are, for example, in a range of 20 to 150 °C. Preferably the reaction temperatures are in a range of 40 to 60 °C. for the IEDDA reaction are in a range of 30 to 70 °C. Most preferably the reaction temperatures for the IEDDA reaction

For the various embodiments, the IEDDA reactions are conducted under an inert atmosphere, where an N₂ atmosphere is one example. The IEDDA reactions can also be conducted under atmosphere air. For the various embodiments, the crystalline porous MOF is suspended in an organic solvent, as provided herein, to which the phosphorous ligand of the present disclosure is added. Preferably, the solvent for the tetrazine based click-grafting reaction can be selected from chlorinated solvents such as dichloromethane, chlorobenzene, 1,2-dichloroethane, 1,1-dichloroethane, chloroform, and mixtures thereof. In addition, preferred solvents possess a moderate dipole moment (e.g., polar solvents, such as dimethylformamide among others). Such solvents can also include mixtures of the above noted solvents with one or more of a protic solvent which can include water.

For the tetrazine based click-grafting reaction, the crystalline porous MOF can be provided in a molar equivalent or molar excess relative the phosphorus ligand. For example, the range of the molar amounts of the tetrazine present in the crystalline porous MOF to the dienophile of the phosphorous ligand can be from 1:1 to 1000:1. Preferably, the range of the molar amounts of the tetrazine present in the crystalline porous MOF to the dienophile of the phosphorous ligand is from 1:1 to 100:1, and more preferably from 1:1 to 13:1, where a value of 10:1 is possible. For the tetrazine based click-grafting reaction, the crystalline porous MOF can be provided in a molar equivalent or molar excess relative the dienophile appended phosphorus ligand. For example, the range of the molar amounts of the tetrazine present in the crystalline porous MOF to the dienophile of the phosphorous ligand can be from 1:1 to 1000:1. Other suitable dienophiles can include an enamine, as are known, where the amount of amine to ketone used in forming the enamine can be from 1000:1 to 1:1, preferably 10:1.

The IEDDA reaction mixture of the crystalline porous MOF and the phosphorus ligand is allowed to react under the inert atmosphere (e.g., an N₂ atmosphere) for a reaction time of 1 day to 5 days at the desired reaction temperature. For example, the IEDDA reaction mixture of the crystalline porous MOF and the phosphorus ligand is heated to the desired reaction temperature (e.g., 25 ^{O}C) under an inert atmosphere for 48 hours (2 days). After the reaction time, the resulting heterogenous catalyst precursor is washed with and subsequently allowed to soak in fresh organic solvent, as provided herein *(e.g.,* dichloromethane), at room temperature (23 ^{O}C). Soaking times for the heterogenous catalyst precursor can be from 30 minutes to 120 minutes. The heterogenous catalyst precursor is then activated at a temperature in a range of 30 to 100 ^{O}C under a high vacuum 0.00001 to 1 KPa for 12 to 24 hours. Preferably, the temperature for activating the heterogenous catalyst precursor can be from 40 to 90 ^{O}C.

In an additional embodiment, tetrazine groups remaining in the heterogenous catalyst precursor can be reacted with norbornene to avoid side reactions or decomposition of any tetrazine groups remaining in the heterogenous catalyst precursor. The treatment is performed by suspending the heterogenous catalyst precursor in an organic solvent, as provided herein (e.g., dichloromethane), with a molar excess of norbornene under an inert atmosphere *(e.g.,* an N₂ atmosphere). The mixture is heated to 40 to 70 °C for 8 to 20 hours. After which time, the heterogenous catalyst precursor is washed with fresh organic solvent (e.g., dichloromethane) and then fresh hexanes, where the heterogenous catalyst precursor is allowed to soak in each wash for at least 1 hour. The heterogenous catalyst precursor is then activated as discussed above.

The following are examples of the click-reaction partner having a strained dienophile, as discussed herein, that can react with the tetrazine moiety in the crystalline porous MOF. Examples provided herein can be didentate or mono-dentate. In one embodiment, the phosphorous ligand can be of Formula II: 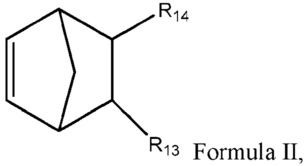 where the IEDDA reaction as discussed herein forms the heterogeneous catalyst precursor of Formula III:

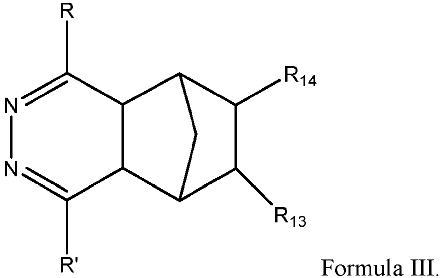

In an alternative embodiment, the phosphorous ligand can be of Formula IV: 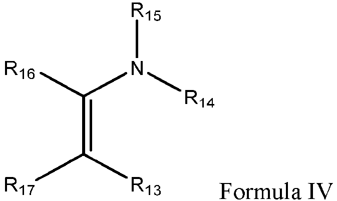 where the IEDDA reaction forms the heterogeneous catalyst precursor of Formula V: 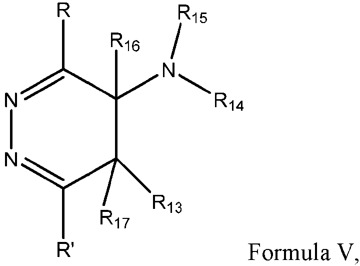 or

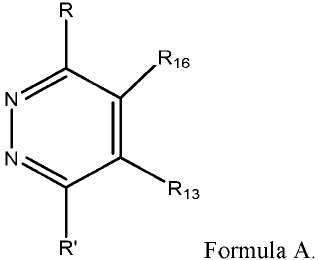

In an additional embodiment, the phosphorous ligand can be of Formula VI: 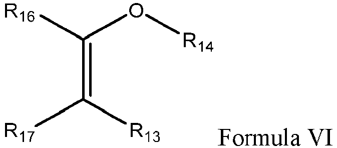 where the IEDDA reaction forms the heterogeneous catalyst precursor of Formula VII 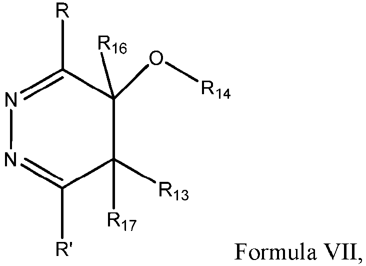 or

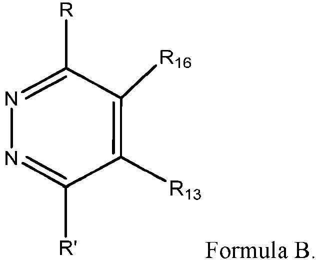

In an additional embodiment, the phosphorous ligand can be of Formula VIII or Formula C: 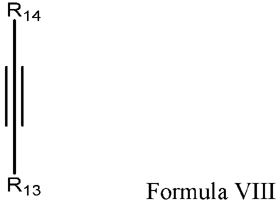 or 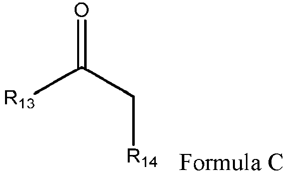 and an amine catalyst , where the reaction forms the heterogeneous catalyst precursor of Formula IX:

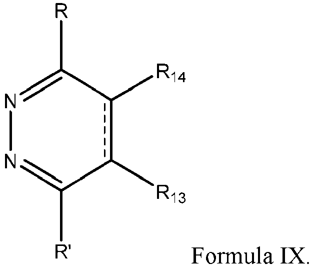

In an additional embodiment, the phosphorous ligand can be of Formula D:

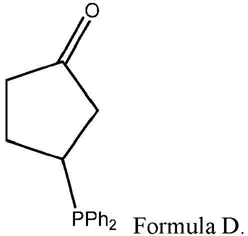

With respect to the use of Formula C, an enamine can be generated *in situ* by condensation of the ketone of Formula C and an appropriate amine catalyst, such as pyrrolidine, according to the following reaction: 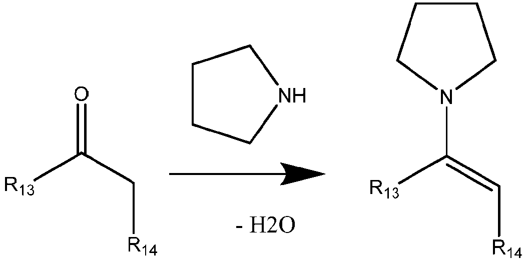 where the IEDDA reaction produces the catalyst precursor of the present disclosure, but eliminates the amine to produce the structure of Formula IX as follows:

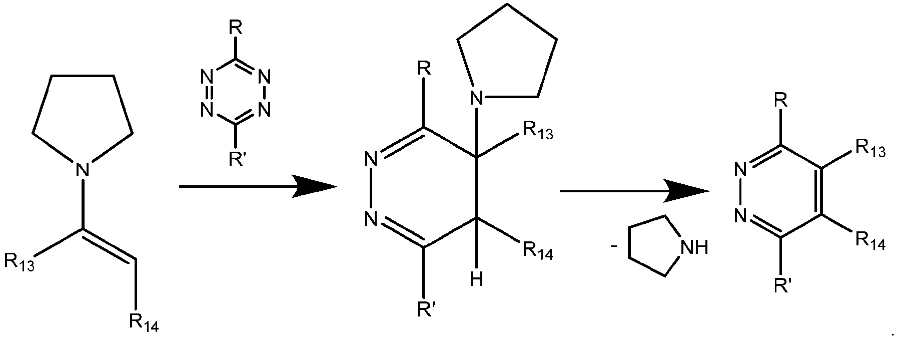

For example, when 3-(diphenylphosphine)cyclopentanone (Formula D) is used, the following reaction according to the present disclosure, is possible:

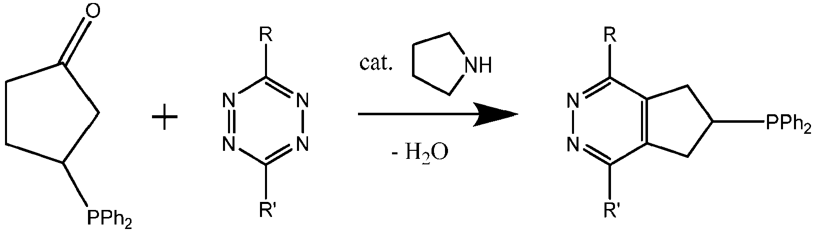

For the above formulae A and II through IX, R₁₆ and R₁₇ are each independently -H and C₁ to C₃ alkyl, and R₁₃ - R₁₅ are each independently selected from - H, C₁ to C₃₀ alkyl and at least one of: 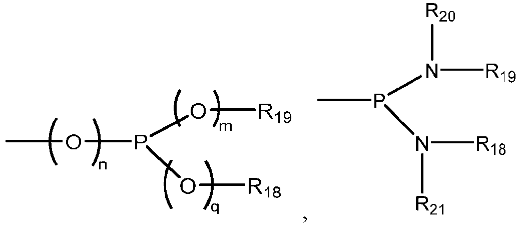 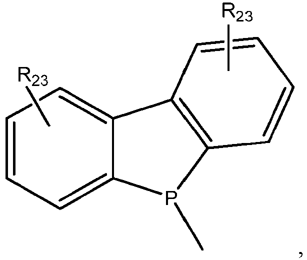 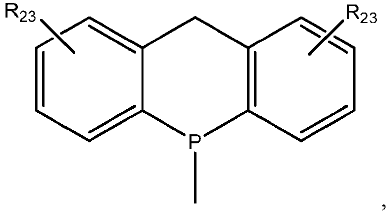 PPh₂ or

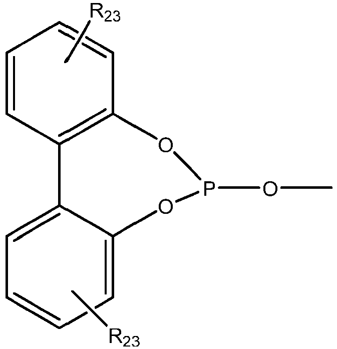

Preferably, for the above formulae II through IX, R₁₅ is selected from -H, C₁ to C₃₀ alkyl and R₁₃ and R₁₄ are each independently selected from: 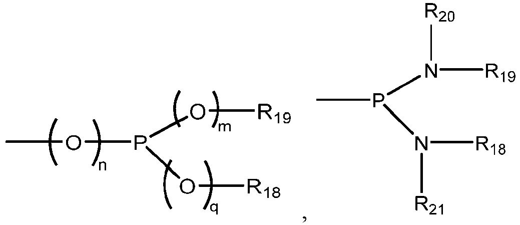 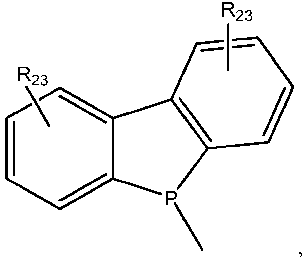 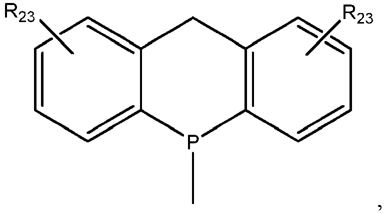 PPh₂ or 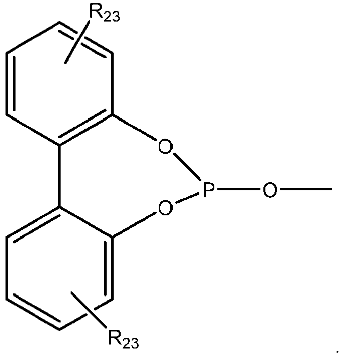 Preferably, R₁₃ and R₁₄ are each the same moiety.

For the various embodiments, each of n, m, and q are either 0 or 1, where for the above moieties the variables (n,m,q) are either (1,0,0), (1,1,0), (0,0,1), (0,1,1) or (1,1,1). For the various embodiments, each of R₁₈ through R₂₁ are selected from C₅-C₁₀ aryl, C₁-C₄ alkyl or -N(R₂₂)₂, where R₂₂ is selected from -H or C₁ to C₃ alkyl. Each of R₂₃ is selected from C₅-C₁₀ aryl, C₁-C₄ alkyl, -OR₂₂ or -N(R₂₂)₂, where R₂₂ is selected from -H or C₁ to C₃ alkyl.

### Heterogeneous Catalyst Composition

The heterogeneous catalyst precursor, as provided herein, can then react with a Group VIII transition metal catalyst precursor compound to form the heterogeneous catalyst composition. As discussed herein, the heterogeneous catalyst composition of the present disclosure can be used in any number of reactions, including hydroformylation, carbonylation of alcohols, cross-coupling and asymmetric hydrogenation, and ethylene oligomerization, among others.

For the various embodiments, the Group VIII transition metal catalyst precursor compound is of Formula X:

M_{w}(L¹)ₓ(L²)_{y}(L³)_{z} Formula X

where the M is selected from the group consisting of rhodium (Rh), cobalt (Co), iridium (Ir), ruthenium (Ru), iron (Fe), nickel (Ni), palladium (Pd), platinum (Pt), and osmium (Os).

For the various embodiments, L¹, L² and L³ are each independently selected from the group consisting of hydrogen, carbonyl (CO), cyclooctadiene, norbornene, chlorine, oxygen, boron, fluoride, bromide, iodide, nitrate, acetate, octanoate, 2-ethylhexanoate, triphenylphosphine (TPP), and acetylacetonate (AcAc).

For Formula X, w is an integer from 1 to 6, and x, y and z are each independently an integer from 0 to 5 wherein the sum of x, y, and z is at least 1.0.

For the various embodiments, the transition metal catalyst precursor of Formula X is selected from the group consisting of Rh(O₂C₅H₇)(CO)₂, Rh₂O₃, Rh₄(CO)₁₂, Rh₆(CO)₁₆, Rh(NO₃)₃, RhCl₃xH₂O, Rh(OAc)₃, Rh(AcAc)(CO)₂, Rh(AcAc)(1,5-COD), Rh(1,5-COD)₂BF₄ Rh(NBD)₂(BF₄), [Rh(1,5-COD)Cl]₂, [Rh(NBD)Cl]₂, tris(triphenylphosphine)rhodium carbonyl hydride, Co₂(CO)₈, cobalt (II) octanoate, Ir(1,5-COD)(AcAc), [Ir(1,5-COD)Cl]₂, Ir(1,5-COD)₂BF₄, Ru3(CO)12, RuCl₃, [HFe(CO)₄]⁻, Ni(OAc)₂, Pd(AcAc)₂, Pd(OTf)₂, PtCl₂(PhCN)₂, OsO₄, or combinations thereof.

For the reaction between the heterogeneous catalyst precursor and the Group VIII transition metal catalyst precursor compound to form the heterogeneous catalyst composition, the heterogeneous catalyst precursor and the Group VIII transition metal catalyst precursor compound are mixed with a solvent. The solvent can be selected from the group consisting of toluene, acetonitrile and dimethylformamide among others. Such solvents can also include mixtures of the above noted solvents.

The reaction is preferably conducted under the inert atmosphere (e.g., an N₂ atmosphere) for a reaction time of 1 hour to 5 days at the given reaction temperature. Preferably, the reaction time is from 24 hours to 48 hours. The reaction can take place at a temperature, for example, in a range of 20 to 150 ^{O}C. Preferably the reaction temperatures is in a range of 20 to 70 ^{O}C. Most preferably the reaction temperature is in a range of 25 to 50 ^{O}C.

Preferably, for the reaction the heterogeneous catalyst precursor is provided in a molar excess (based on phosphorous content) relative the Group VIII transition metal catalyst precursor compound, where phosphorous content can be measured by known analytical methods or could be on a mass balance basis. For example, the heterogeneous catalyst precursor is provided in a molar excess of 1:0.001 to 1:0.25 relative to the Group VIII transition metal catalyst precursor compound. Preferably, the heterogeneous catalyst precursor is provided in a molar excess of 1:0.01 to 1:0.25 relative to the Group VIII transition metal catalyst precursor compound. More preferably, the heterogeneous catalyst precursor is provided in a molar excess of 1:0.1 to 1:0.25 relative to the Group VIII transition metal catalyst precursor compound. Most preferably, the heterogeneous catalyst precursor is provided in a molar excess of 1:0.1 to 1:0.2 relative to the Group VIII transition metal catalyst precursor compound. Such a molar excess of the Group VIII transition metal catalyst precursor compound relative to the heterogeneous catalyst precursor better ensures that the Group VIII transition metal catalyst precursor compound is bound to the sites of the heterogeneous catalyst precursor, where any free Group VIII transition metal catalyst precursor compounds are more likely to bind or be re-bound to the heterogeneous catalyst precursor.

After the reaction time, the resulting heterogenous catalyst precursor is washed with and subsequently allowed to soak in fresh organic solvent at room temperature (23 ^{O}C). Soaking times for the heterogenous catalyst precursor can be from 30 minutes to 120 minutes. The heterogenous catalyst precursor is then activated at a temperature in a range of 30 to 100 ^{O}C under a high vacuum 0.00001 to 1 Pa for 12 to 24 hours. Preferably, the temperature for activating the heterogenous catalyst precursor can be from 40 to 90 ^{O}C.

### Reactions and Reactors

As provided herein, the heterogeneous catalyst composition of the present disclosure may be useful in a broad classes of reactions such as hydroformylation, carbonylation of alcohols, cross-coupling and asymmetric hydrogenation, and olefin oligomerization, among other reaction provided here. Other illustrative reactions include, for example, hydroacylation (intramolecular and intermolecular), hydrocyanation, hydroamidation, hydroesterification, aminolysis, alcoholysis, hydrocarbonylation, olefin isomerization, transfer hydrogenation and the like. Preferred processes involve the reaction of organic compounds with carbon monoxide, or with carbon monoxide and a third reactant, e.g., hydrogen, or with hydrogen cyanide, in the presence of a catalytic amount of the heterogeneous catalyst composition of the present disclosure. The most preferred processes include hydroformylation, hydrocyanation, hydrocarbonylation, hydroxycarbonylation and carborlylation.

The permissible starting material reactants encompassed by the processes provided herein are, of course, chosen depending on the particular process desired. Such starting materials are well known in the art and can be used in conventional amounts in accordance with conventional methods. Illustrative starting material reactants include, for example, substituted and unsubstituted aldehydes, (intramolecular hydroacylation), olefins (hydroformylation, carbonylaltion, intermolecular hydroacylation, hydrocyanation, hydroamidation, hydroesterification, aminolysis, alcoholysis), ketones (transfer hydrogenation), epoxides (hydroformylation, hydrocyanation), alcohols (carbonylation) and the like. Illustrative of suitable reactants for effecting the processes of this disclosure are set out in Kirk-Othmer, Encyclopedia of Chemical Technology, Fifth Edition, 2004.

A preferred process useful with the heterogeneous catalyst composition of the present disclosure is hydroformylation. The hydroformylation processing techniques may correspond to known processing techniques. The hydroformylation products may be asymmetric, non-asymmetric or a combination thereof, with the preferred products being non-asymmetric. The process may be conducted in a batch, a continuous or semi-continuous fashion and in some embodiments, involve a catalyst liquid recycle operation. Processes can also include those involving catalyst liquid recycle hydroformylation processes. **In** general, such catalyst liquid recycle hydroformylation processes involve the production of aldehydes by reacting an olefinic unsaturated compound with carbon monoxide and hydrogen in the presence of the heterogeneous catalyst composition of the present disclosure in a liquid medium that also contains a solvent for the catalyst and ligand. Preferably free heterogeneous catalyst precursor can be present in the liquid hydroformylation reaction medium. The recycle procedure generally involves withdrawing a portion of the liquid reaction medium containing the heterogeneous catalyst composition and aldehyde product from the hydroformylation reactor *(i.e.,* reaction zone), either continuously or intermittently, and recovering the aldehyde product therefrom in accordance with known separation techniques. Such techniques can include, but are not limited to, sedimentation, filtration, membrane and/or centrifugation separation processes for separating the heterogeneous catalyst composition from the reaction mixture after producing the aldehyde.

The activity of heterogeneous catalyst composition, as quantified using reaction rate, is typically measured as the number of moles of product (aldehyde) per volume of catalyst solution per unit of time (generally scaled by the concentration of active metal) with units of gmols of aldehyde/L/hr/ppm Rh, for example. Other conventional measurements are in units of "turn-over number" or TON (units of hr⁻¹), which is moles of aldehyde produced per moles of heterogeneous catalyst composition. The moles of heterogeneous catalyst composition are typically determined by measuring the amount of metal (e.g., rhodium) in ppm using Atomic Absorption or Inductively Coupled Plasma analysis) and comparison to the activity (reaction rate) of a fresh heterogeneous catalyst composition solution to calculate a "% Active" value.

In a preferred embodiment, the hydroformylation reaction mixtures employable herein includes any mixture derived from any corresponding hydroformylation process that contains at least some amount of the main ingredients or components, *i.e.,* the aldehyde product, the heterogeneous catalyst composition, the heterogeneous catalyst precursor and an organic solubilizing agent, *e.g.,* a polar solvent. It is to be understood that the hydroformylation reaction mixture compositions employable herein can and normally will contain minor amounts of additional ingredients such as those which have either been deliberately employed in the hydroformylation process or formed *in situ* during the process. Examples of such ingredients that can also be present include unreacted olefin starting material, carbon monoxide and hydrogen gases, and *in situ* formed type products, such as saturated hydrocarbons and/or unreacted isomerized olefins corresponding to the olefin starting materials, and high boiling liquid aldehyde condensation byproducts, as well as other inert co-solvent, e.g., nonpolar solvent, type materials or hydrocarbon additives, if employed.

The substituted or unsubstituted olefin reactants that may be employed in the hydroformylation processes (and other suitable processes) of this disclosure include both optically active (prochiral and chiral) and non-optically active (achiral) olefinic unsaturated compounds containing from 2 to 40, preferably 2 to 20, carbon atoms. Such olefinic unsaturated compounds can be terminally or internally unsaturated and be of straight chain, branched chain or cyclic structures, as well as olefin mixtures, such as obtained from the oligomerization of propene, butene, isobutene, etc. (such as so called dimeric, trimeric or tetrameric propylene and the like, as disclosed, for example, in U.S. Pat. Nos. 4,518,809 and 4,528,433). Moreover, such olefin compounds may further contain one or more ethylenic unsaturated groups, and of course, mixtures of two or more different olefinic unsaturated compounds may be employed as the starting material if desired. For example, commercial alpha-olefins containing four or more carbon atoms may contain minor amounts of corresponding internal olefins and/or their corresponding saturated hydrocarbon and that such commercial olefins need not necessarily be purified from same prior to being reacted. Illustrative mixtures of olefinic starting materials that can be employed in the hydroformylation reactions include, for example, mixed butenes, e.g., Raffinate I and II. Further such olefinic unsaturated compounds and the corresponding products derived therefrom may also contain one or more groups or substituents which do not unduly adversely affect the processes of this disclosure such as described, for example, in U.S. Pat. Nos. 3,527,809, 4,769,498 and the like.

The heterogeneous catalyst composition of the present disclosure may also be useful for the production of non-optically active aldehydes, by hydroformylating achiral alpha-olefins containing from. 2 to 30, preferably 2 to 20, carbon atoms, and achiral internal olefins containing from 2 to 20 carbon atoms as well as starting material mixtures of such alpha olefins and internal olefins.

Illustrative olefins include, for example, ethylene, propylene, 1-butene, 1-pentene, 1-hexene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 2-butene, 2-methyl propene (isobutylene), 2-methylbutene, 2-pentene, 2-hexene, 3-hexane, 2-heptene, 2-octene, cyclohexane, propylene dimers, propylene trimers, propylene tetramers, butadiene, piperylene, isoprene, 2-ethyl-1-hexene, styrene, 4-methyl styrene, 4-isopropyl styrene, 4-tert-butyl styrene, alpha-methyl styrene, 4-tert-butyl-alpha-methyl styrene, 1,3-diisopropenylbenzene, 3-phenyl-1-propene, 1,4-hexadiene, 1,7-octadiene, 3-cyclohexyl-1-butene, and the like, as well as, 1,3-dienes, butadiene, pentenoic acids and salts, *e.g.,* salts of 3- and 4-pentenoic acids, alkyl alkenoates, *e.g.,* methyl pentenoate, alkenyl alkanoates, alkenyl alkyl ethers, alkenols, *e.g.,* pentenols, alkenals, *e.g.,* pentenals, and the like, such as allyl alcohol, allyl butyrate, hex-1-en-4-ol, oct-1-en-4-ol, vinyl acetate, allyl acetate, 3-butenyl acetate, vinyl propionate, vinyl functional silanes, vinyl functional siloxanes, allyl propionate, methyl methacrylate, vinyl ethyl ether, vinyl methyl ether, allyl ethyl ether, n-propyl-7-octenoate, 3-butenenitrile, 5-hexenamide, eugenol, iso-eugenol, safrole, iso-safrole, 4-allylanisole, indene, limonene, beta-pinene, dicyclopentadiene, cyclooctadiene, camphene, linalool, and the like.

Illustrative prochiral and chiral olefins useful in the asymmetric hydroformylation processes (and other asymmetric processes) that can be employed to produce enantiomeric product mixtures that may be encompassed by in this disclosure include those represented by the formula: 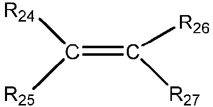 wherein R₂₄, R₂₅, R₂₆ and R₂₇ are the same or different (provided R₂₄ is different from R₂₅ or R₂₆ is different from R₂₇) and are selected from hydrogen, alkyl; substituted alkyl, the substitution being selected from dialkylamino such as benzylamino and dibenzylamino, alkoxy such as methoxy and ethoxy, acyloxy such as acetoxy, halo, nitro, nitrile, thio, carbonyl, carboxamide, carboxaldehyde, carboxyl, carboxylic ester; aryl including phenyl; substituted aryl including phenyl, the substitution being selected from alkyl, amino including alkylamino and dialkylamino such as benzylamino and dibenzylamino, hydroxy, alkoxy such as methoxy and ethoxy, acyloxy such as acetoxy, halo, nitrile, nitro, carboxyl, carboxaldehyde, carboxylic ester, carbonyl, and thio; acyloxy such as acetoxy; alkoxy such as methoxy and ethoxy; amino including alkylamino and dialkylamino such as benzylamino and dibenzylamino; acylamino and diacylamino such as acetylbenzylamino and diacetylamino; nitro; carbonyl; nitrile; carboxyl; carboxamide; carboxaldehyde; carboxylic ester; silane and substituted silane; siloxanes and substituted siloxanes; and alkylmercapto such as methylmercapto. It is understood that the prochiral and chiral olefins of this definition also include molecules of the above general formula where the R groups are connected to form ring compounds, e.g., 3-methyl-1-cyclohexane, and the like.

Illustrative optically active or prochiral olefinic compounds useful in asymmetric hydroformylation processes (and other asymmetric processes) include, for example, p-isobutylstyrene, 2-vinyl-6-methoxy-2-naphthylene, 3-ethenylphenyl phenyl ketone, 4-ethenylphenyl-2-thienylketone, 4-ethenyl-2-fluorobiphenyl, 4-(1,3-dihydro-1-oxo-2H-isoindol-2-yl)styrene, 2-ethenyl-5-benzoylthiophene, 3-ethenylphenyl phenyl ether, propenylbenzene, isobutyl-4-propenylbenzene, phenyl vinyl ether and the like. Other olefinic compounds include substituted aryl ethylenes as described, for example, in U.S. Pat. Nos. 4,329,507, 5,360,938 and 5,491, 266.

Illustrative vinyl functional silanes and vinyl functional siloxanes include trimethoxy(vinyl)silane, dimethoxy(methyl)(vinyl)silane, triethoxy(vinyl)silane, diethoxy(methyl)(vinyl)silane, hex-5-en-1-yltrimethoxysilane, trimethyl(vinyl)silane, 1,1,1,3,5,5,5-heptamethyl-3-vinyltrisiloxane, 1,1,3,3-tetramethyl-1,3-divinyldisiloxane.

Illustrative of suitable substituted and unsubstituted olefinic starting materials include those permissible substituted and unsubstituted olefinic compounds described in Kirk-Othmer, Encyclopedia of Chemical Technology, Fifth Edition, 2004.

The hydroformylation processes involves the use of the heterogeneous catalyst composition of the present disclosure. Mixtures of such heterogeneous catalyst compositions can be employed if desired. The amount of the heterogeneous catalyst composition present in the reaction medium of a given hydroformylation process can include, for example, that minimum amount necessary to provide the given metal concentration desired to be employed and which will furnish the basis for at least the catalytic amount of metal necessary to catalyze the particular hydroformylation process. **In** general, metal, e.g., rhodium, concentrations in the range of from about 10 ppm to about 1000 ppm, calculated as rhodium, in the hydroformylation reaction medium should be sufficient for most processes, while it is generally preferred to employ from about 100 to 500 ppm of metal, e.g., rhodium, and more preferably from 250 to 400 ppm of metal, e.g., rhodium. Analytical techniques for measuring catalytic metal concentrations are well known to the skilled person, and include atomic absorption (AA), inductively coupled plasma (ICP) and X-ray fluorescence (XRF); AA is typically preferred.

In addition to the heterogeneous catalyst composition, free heterogeneous catalyst precursor *(i.e.,* heterogeneous catalyst precursor that is not complexed with the Group VIII transition metal catalyst precursor compound to form the heterogeneous catalyst composition) may also be present in the hydroformylation reaction medium. The free heterogeneous catalyst precursor may correspond to any of the above-defined heterogeneous catalyst precursor. It is preferred that the free heterogeneous catalyst precursor be the same as the heterogeneous catalyst precursor used in forming the heterogeneous catalyst composition. However, such heterogeneous catalyst precursor need not be the same in any given process. The hydroformylation process may involve from about 0.1 moles or less to about 100 moles or higher of free heterogeneous catalyst precursor, based on phosphorous content, per mole of metal in the hydroformylation reaction medium.

The reaction conditions of the hydroformylation processes may include any suitable type hydroformylation conditions known in the art for producing optically active and/or non-optically active aldehydes. For instance, the total gas pressure of hydrogen, carbon monoxide and olefin starting compound of the hydroformylation process may range from about 1 to 69,000 kilopascal (kPa). In general, however, it is preferred that the process be operated at a total gas pressure of hydrogen, carbon monoxide and olefin starting compound of less than 14,000 kPa and more preferably less than 3,400 kPa. The minimum total pressure is limited predominantly by the amount of reactants necessary to obtain a desired rate of reaction. More specifically, the carbon monoxide partial pressure of the hydroformylation process is preferably from 1 to 6,900 kPa, and more preferably from 21 to 5,500 kPa, while the hydrogen partial pressure is preferably from 34 to 3,400 kPa and more preferably from 69 to 2,100 kPa. In general, H₂:CO molar ratio of gaseous hydrogen to carbon monoxide may range from about 1:10 to 100:1 or higher, the more preferred hydrogen to carbon monoxide molar ratio being from about 1:10 to about 10:1.

Further, the hydroformylation process, may be conducted at a reaction temperature from about -25 ^{O}C to about 200 ^{O}C. In general, hydroformylation reaction temperatures of about 50 ^{O}C to about 120 ^{O}C are preferred for all types of olefinic starting materials. When non-optically active aldehyde products are desired, achiral type olefin starting materials and organophosphorus ligands are employed and when optically active aldehyde products are desired prochiral or chiral type olefin starting materials and organophosphorus ligands are employed. It is to be also understood that the hydroformylation reaction conditions employed will be governed by the type of aldehyde product desired.

A solvent advantageously is employed in the hydroformylation process. Any suitable solvent that does not unduly interfere with the hydroformylation process can be used. The organic solvent may also contain dissolved water up to the saturation limit. When the metal of the heterogeneous catalyst composition is rhodium, it may be preferred to employ, as a primary solvent, aldehyde compounds corresponding to the aldehyde products desired to be produced and/or higher boiling aldehyde liquid condensation by-products, for example, as might be produced *in situ* during the hydroformylation process, as described for example in U.S. Pat. Nos. 4,148,830 and 4,247,486. Indeed, while one may employ, if desired, any suitable solvent at the start-up of a continuous process, the primary solvent will normally eventually comprise both aldehyde products and higher boiling aldehyde liquid condensation by-products ("heavies"), due to the nature of the continuous process. The amount of solvent need only be sufficient to provide the reaction medium with the desired amount of transition metal concentration. Typically, the amount of solvent ranges from about 5 percent to about 95 percent by weight, based on the total weight of the reaction fluid. Mixtures of two or more solvents may also be employed.

Illustrative non-optically active aldehyde products include *e.g.,* propionaldehyde, n-butyraldehyde, iso-butyraldehyde, n-valeraldehyde, 2-methyl 1-butyraldehyde, hexanal, hydroxyhexanal, 2-methyl valeraldehyde, helitanal, 2-methyl 1-hexanal, octanal, 2-methyl 1-heptanal, nonanal, 2-methyl-1-octanal, 2-ethyl 1-heptanal, 3-propyl 1-hexanal, decanal, adipaldehyde, 2-methylglutaraldehyde, 2-methyladipaldehyde, 3-methyladipaldehyde, 3-hydroxypropionaldehyde, 6-hydroxyhexanal, alkenals, *e.g.,* 2-, 3- and 4-pentenal, formylvaleric acids and salts, e.g., salts of 5-formylvaleric acid, alkyl 5-formylvalerate, 2-methyl-1-nonanal, undecanal, 2-methyl 1-decanal, dodecanal, 2-methyl 1-undecanal, tridecanal, 2-methyl 1-tridecanal, 2-ethyl, 1-dodecanal, 3-propyl-1-undecanal, pentadecanal, 2-methyl-1-tetradecanal, hexadecanal, 2-methyl-1-pentadecanal, heptadecanal, 2-methyl-1-hexadecanal, octadecanal, 2-methyl-1-heptadecanal, nonodecanal, 2-methyl-1-octadecanal, 2-ethyl 1-heptadecanal, 3-propyl-1-hexadecanal, eicosanal, 2-methyl-1-nonadecanal, heneicosanal, 2-methyl-1-eicosanal, tricosanal, 2-methyl-1-docosanal, tetracosanal, 2-methyl-1-tricosanal, pentacosanal, 2-methyl-1-tetracosanal, 2-ethyl 1-tricosanal, 3-propyl-1-docosanal, heptacosanal, 2-methyl-1-octacosanal, nonacosanal, 2-methyl-1-octacosanal, hentriacontanal, 2-methyl-1-triacontanal, and the like.

Illustrative optically active aldehyde products include (enantiomeric) aldehyde compounds prepared by the asymmetric hydroformylation process such as, *e.g.* S-2-(p-isobutylphenyl)-propionaldehyde, S-2-(6-methoxy-2-naphthyl)propionaldehyde, S-2-(3-benzoylphenyl)-propionaldehyde, S-2-(p-thienoylphenyl)propionaldehyde, S-2-(3-fluoro-4-phenyl)phenylpropionaldehyde, S-2- [4-(1,3-dihydro-1-oxo-2H-isoindol-2-yl)phenyl]propionaldehyde, S-2-(2-methylacetaldehyde)-5-benzoylthiophene and the like.

Illustrative aldehyde functional silanes/siloxanes products include 3-(trimethoxysilyl)propanal, 3-(dimethoxy(methyl)silyl)propanal, 3-(triethoxysilyl)propanal, 3-(diethoxy(methyl)silyl)propanal, 7-(trimethoxysilyl)heptanal, 3-(trimethylsilyl)propanal, 3-(1,1,1,3,5,5,5-heptamethyltrisiloxan-3-yl)propanal, 3,3'-(1,1,3,3-tetramethyldisiloxane-1,3-diyl)dipropanal.

Illustrative of suitable substituted and unsubstituted aldehyde products include those permissible substituted and unsubstituted aldehyde compounds described in Kirk-Othmer, Encyclopedia of Chemical Technology, Fifth Edition, 2004.

The aldehyde product mixtures may be extracted and separated from the other components of the crude reaction mixtures in which the aldehyde mixtures are separated by techniques as discussed herein. It is generally preferred to carry out the hydroformylation processes in a continuous manner. **In** general, continuous hydroformylation processes are well known in the art and may involve: (a) hydroformylating the olefinic starting material(s) with carbon monoxide and hydrogen in a liquid homogeneous reaction mixture comprising a polar solvent, the heterogeneous catalyst composition, free heterogeneous catalyst precursor, and optionally a nonpolar solvent; (b) maintaining reaction temperature and pressure conditions favorable to the hydroformylation of the olefinic starting material(s); (c) supplying make-up quantities of the olefinic starting material(s), carbon monoxide and hydrogen to the reaction medium as those reactants are used up; (d) mixing at least a portion of the reaction medium with a nonpolar solvent to extract the desired aldehyde hydroformylation product(s) from the reaction medium; and (e) recovering the desired aldehyde product(s) by, for example, phase separation.

At the conclusion of (or during) the hydroformylation process, the desired aldehydes may be recovered from the reaction mixtures. For instance, in a continuous liquid catalyst recycle process the portion of the liquid reaction mixture (containing the aldehyde product) can be removed from the reaction zone and passed to a separation zone where the desired aldehyde product can be extracted and separated via phase separation from the liquid reaction mixture, and further purified if desired. The remaining catalyst containing liquid reaction mixture may then be recycled back to the reaction zone as may if desired any other materials, e.g., unreacted olefin, together with all hydrogen and carbon monoxide dissolved in the liquid reaction after separation thereof from the aldehyde product.

The hydroformylation process of this disclosure may be carried out using one or more suitable reactors such as, for example, a fixed bed reactor, a tubular reactor, a venturi reactor, a bubble column reactor, a continuous stirred tank reactor (CSTR) or a slurry reactor, with the recycle of unconsumed starting materials. The optimum size and shape of the reactor will depend on the type of reactor used. The at least one reaction zone employed in this disclosure may be a single vessel or may comprise two or more discrete vessels in series or in parallel. The separation zone employed may be a single vessel or may comprise two or more discrete vessels. The buffer treatment zone employed in this disclosure may be a single vessel or may comprise two or more discreet vessels. The reaction zone(s) and separation zone(s) employed herein may exist in the same vessel or in different vessels. For example, reactive separation techniques such as reactive distillation, reactive membrane separation, and the like, may occur in the reaction zone(s).

The materials of construction employed should be substantially inert to the starting materials during the reaction and the fabrication of the equipment should be able to withstand the reaction temperatures and pressures. Means to introduce and/or adjust the quantity of starting materials or ingredients introduced continuously into the reaction zone during the course of the reaction can be conveniently utilized in the process especially to maintain the desired molar ratio of the starting materials. The starting materials and/or recycled olefin may be added to each or all the reaction zones in series.

The hydroformylation process may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

The hydroformylation process of this disclosure may be conducted in one or more steps or stages. The exact number of reaction steps or stages will be governed by the best compromise between capital costs and achieving high catalyst selectivity, activity, lifetime and ease of operability, as well as the intrinsic reactivity of the starting materials in question and the stability of the starting materials and the desired reaction product to the reaction conditions.

For the various embodiments, the present disclosure further includes separating the heterogeneous catalyst composition from the reaction mixture after producing the aldehyde using one of a decanting, filtration process, a membrane separation process or a centrifuge separation process. A catalyst basket can also be used to hold the heterogeneous catalyst composition during the reaction, which can then be lifted from the reaction mixture directly. **In** addition, if the substrate, products, and solvent of the reaction mixture are sufficiently volatile, the heterogeneous catalyst composition can be recycled directly in the reactor. For example, this could be done by purging the reactor of syngas and then applying a vacuum sufficient to evaporate the liquid components, which would be collected in a N₂ trap. Alternatively, the heterogeneous catalyst composition could be filtered and a backflush of fresh reagents/solvents could be used to re-introduce the heterogeneous catalyst composition back into the reactor, Such techniques would allow for the heterogeneous catalyst composition to be isolated for recycling without exposure to air or water.

The heterogeneous catalyst composition recovered from the reaction mixture can be recycled by a series of trituration and soaking steps. For example, the heterogeneous catalyst composition can be triturated several times (e.g., three times) with a wash solvent (fresh each time) at room temperature (e.g., 23 ^{O}C) followed by a soak in the wash solvent for approximately one hour (h). The wash solvent is then replaced with fresh wash solvent and the heterogeneous catalyst composition soaked again for 1 h. The heterogeneous catalyst composition is then triturated three time with hexanes (C₆H₁₄), then soaked once in fresh hexanes for 1 h, and then finally the solvent is decanted and the solids dried in vacuo to yield the recycled heterogeneous catalyst composition. Suitable wash solvents include toluene or diethyl ether. The use of other wash solvents and organic solvents besides hexanes is also possible. In addition, soak times can range from thirty (30) minutes to several days. A soak time of one hour, however, is typically sufficient.

### EXAMPLES

### Materials

The following materials were used in the following Examples (Ex) and Comparative Examples (CE). All materials were purchased from the following companies and used as received, unless otherwise noted.

4-bromo-3-trifluoromethylbenzonitrile; PdCl₂(PPh₃)₂; 4-bromo-3-fluorobenzonitrile; and 1-Boc-pyrazole-4-boronic acid pinacol ester from Matrix Scientific.

Rh(acac)(CO)₂, benzoic acid; 1-octene; pyrrolidine; NaNO₃; lithium formate; hydrazine monohydrate; formic acid; sulfur (S₈); allyldiphenylphosphine; trichlorosilane; Na₂CO₃, Mg(NO₃)₂[H₂O]₆; MgSO₄; 1-chlorobutane; benzoquinone; ZrCl₄; and AuCl from Sigma-Aldrich^{®}.

Hydroxylamine HCl from Alfa Aesar.

2-norbornene; dicyclopentadiene; 4-cyano-2-fluorobenzoic acid; 1-nonanal; and 5-formylsalicylic acid from TCI^{®} (Tokyo Chemical Industry).

(R,R)-(-)-Norphos (Norphos) and vinyldiphenylphosphine from Strem Chemicals.

N-acetyl-L-cysteine from Oakwood Chemical.

DMSO-d₆ and D₂SO₄ from Cambridge Isotope Laboratories, Inc.

Synthesis gas (1:1 mixture of H₂/CO) from AirGas^{®}.

*N*,*N*-Dimethylformamide (DMF); ethanol (EtOH); methanol (MeOH); dimethoxyethane (DME); ethyl acetate (EtOAc); dichloromethane (DCM); diethyl ether (Et₂O); and anhydrous toluene from Sigma-Aldrich^{®}? were of standard grade and used as received without further purification.

Ni(OAc)₂(H₂O)₄ from Strem.

H₂O₂ solution, 10 percent weight/weight (H₂O₂ 10% w/w) from Sigma-Aldrich^{®}.

Petroleum ether from Sigma-Aldrich^{®}.

Hydrazine monohydrate from TCI.

NaNO₂ from Sigma-Aldrich^{®}.

Glacial AcOH from Sigma-Aldrich^{®}.

DI Water (H₂O).

Dry, deaerated MeOH, DME, and DCM were obtained by passing the solvent through two silica columns in a Glass Contour Solvent System and degassing with a flow of argon gas for thirty minutes (min), followed by three freeze-pump-thaw cycles. Cyclopentadiene was obtained by cracking dicyclopentadiene in hot decalin immediately prior to use. All other commercial reagents were used as received without further purification.

All BET Surface Area measurements for the following examples were conducted using a ASAP 2020 adsorption analyzer (Micromeritics^{®}) with nitrogen used as the analysis gas.

As used herein, the @ symbol represents the covalent bonds used to join the identified species onto the MOF framework.

### Synthesis of Phosphorus Ligand 5-diphenylphosphinobicyclo[2.2.1]hept-5-ene (Norl)

In a 100 milliliter (mL) round-bottom flask, vinyl diphenyl phosphine (1.00 gram, g) was dissolved in DCM (8 mL, stabilizer free), followed by addition of H₂O₂ 10% w/w (8 mL) to form a biphasic solution. The biphasic solution was stirred overnight at room temperature (23 ^{O}C). The following day, DI water (50 mL) was added to the stirring biphasic solution after which the stirring was stopped and the layers partitioned in a separatory funnel. The organic phase was collected, dried with Na₂SO₄, filtered over a fritted glass funnel , and the volatiles removed under vacuum to yield the vinyl diphenyl phosphine oxide as white solids.

The vinyl diphenyl phosphine oxide (0.912 g) was combined with cyclopentadiene (4 mL) and anhydrous toluene (15 mL) in a heated and stirred reactor (Parr stirred reactor model Series 4760 100 mL), which was then sealed and heated to 160 °C for two to three hours (h) with stirring. The reactor was allowed to cool to room temperature, then the reactor opened and contents transferred to a 25 mL round bottom flask. The volatiles were removed under vacuum, then petroleum ether added to precipitate the racemic norbornyl phosphine oxide as white solids. The solids were collected on a frit, further washed with petroleum ether, then the solids dried in vacuo and transferred to a glovebox under dry nitrogen (N₂ glovebox).

Under dry nitrogen, a Parr bomb reactor (Parr reactor model Series 4760 100 mL) was charged with the racemic norbornyl phosphine oxide, trichlorosilane (300 microliters, µL), and anhydrous toluene (15 mL). The Parr bomb reactor was sealed, removed from the N₂ glovebox, and transferred to an oil bath heated to 100 °C for 1 h. The Parr bomb reactor was then returned to the N₂ glovebox. The contents of the Parr bomb reactor were transferred to a 50 mL round-bottom flask and the volatiles removed under vacuum. The residue was triturated with - 35 °C MeOH (3x1 mL), filtered, and finally dried to yield 5-diphenylphosphinobicyclo[2.2.1]hept-5-ene as a mixture of endo and exo isomers (25-38% yield). Mixture of exo and endo isomers were measured using the integration ratio of the ³¹P-NMR peaks, in approximate 1:1.6 ratio. Due to the complicated and overlapping nature of the 1H spectrum, chemical shifts and multiplicities are reported, but are not assigned to each isomer. The results were as follows: ¹H-NMR (DMSO d6, 400 MHz ). δ 7.64 - 6.87 (multiple peaks), 6.16-6.10 (multiple peaks), 2.95 - 2.81 (multiple peaks), 2.65 (s), 2.56 (t, *J =* 3.9 Hz), 2.18 - 2.10 (m, 1H), 1.97 (m, 1H), 1.69 (d, *J* = 8.2 Hz), 1.46 - 1.18 (multiple peaks), 0.99 (dt, *J =* 16.9, 7.5 Hz), 0.85 - 0.64 (td, *J =* 10.1, 4.0 Hz ); ³¹P-NMR (DMSO-d6, 25 °C, 162MHz). δ -4.18, -6.41.

### Synthesis of Crystalline Porous MOF Ni₈-tet-F

### 3-fluoro-4-pyrazolylbenzonitrile.

A 1L 3-neck round-bottom flask was charged with 4-bromo-3-fluorobenzonitrile (5.74 g, 28.7 mmol), 1-Boc-pyrazole-4-boronic acid pinacol ester (12.5 g, 42.5 mmol), PdCl₂(PPh₃)₂ (1.375 g, 1.96 mmol), Na₂CO₃ (8.22 g, 78.0 mmol) and a 1" stir bar, then the flask was affixed with two stoppers and a condenser. The system was then evacuated and backfilled with nitrogen (N₂) three times.

While stirring, DME (175 mL, dry & deaerated) was added to the flask via cannula, followed by H₂O (47.5 mL, vigorously sparged with N₂). The reaction was then heated to reflux (approximately 85 ^{O}C) in an oil bath and vigorously stirred overnight. After heating, the reaction was removed from the oil bath and allowed to cool to room temperature, then quenched by adding H₂O (200 mL) and EtOAc (375 mL) together in one portion.

The reaction mixture was partitioned in a 1 L separatory funnel and the organic phase was collected. The aqueous phase was then washed with EtOAC (3 x 100 mL). The organic extractions were combined, washed with brine (saturated aqueous sodium chloride, 125 mL), dried over MgSO₄, and filtered. The volatiles were reduced to about 100 mL in vacuo, then hexanes (500 mL) added in one portion to precipitate yellow solids. The solids were collected on a frit and washed with hexanes (3 x 50 mL), then ice cold MeOH (2 x 10 mL), and finally dried under vacuum to yield 3-fluoro-4-pyrazolylbenzonitrile (2.90 g pale yellow solids, 15.5 mmol, 54% yield). ¹H NMR (400 MHz, DMSO-d6). δ 13.31 (br s, 2H, pyrazole N-H), 8.34 (s, 1H, pyrazole C-H), 8.07 (s, 2H, pyrazole C-H), 7.98 (t, 1 H, aryl C-H), 7.89 (m, 1H, aryl C-H), 7.70 (m, 1H, aryl C-H) ppm. ¹⁹F-NMR (376.5 MHz, DMSO-d6). δ -112.63 ppm (s, 1F, aryl C-F).

### 1,4-bis[1-(4-pyrazolyl-)-2-fluorophenyl]tetrazine (BPAT-F)

In a 20 mL scintillation vial, 3-fluoro-4-pyrazolylbenzonitrile (562 mg, 3.00 mmol) was suspended in ethanol (4.5 mL). A ½" stir bar was added to the vial. Slowly, with stirring, and under a gentle stream of N₂, sulfur (101 mg, 0.394 mmol) was added, followed by slow addition of hydrazine monohydrate (0.70 mL, 720 mg, 14 mmol). Upon addition of the hydrazine monohydrate, the suspension rapidly darkened to a deep red-brown and the solids dissolved to form a homogeneous solution. The vial was tightly capped and carefully heated to 65 °C for 4 h, during which the dihydrotetrazine precipitated from solution as orange solids.

After heating, the vial was allowed to cool to room temperature. The cooled vial was carefully opened inside of a nitrile glove to protect from spraying. The reaction mixture was diluted with H₂O (4 mL). The orange solids were collected on a frit, washed with MeOH until the filtrate was colorless, and fully dried under vacuum.

In a 250 mL Erlenmeyer flask, the dried dihydrotetrazine was suspended in H₂O (75 mL) and sonicated until a fine and chunk-free suspension was formed. A 1" stir bar was added to the flask. With rapid stirring, NaNO₂ (1.6 g, 23 mmol) was added to the flask, followed by addition of glacial AcOH (100 mL). Upon addition of the AcOH, the suspension rapidly changes in color from orange to rust-red over about 30 s. An additional portion of AcOH (50 mL) was added, and the suspension stirred for 1 h at room temperature. The solids were then collected on a large frit and washed with copious H₂O, followed by DMF (2 x 10 mL). The solid cake was agitated between each wash and the washes were filtered to dampness. The product was fully dried on the frit to yield BPAT-F (190 mg rust-red solids, 0.48 mmol, 32% yield). ¹H NMR (400 MHz, neat D₂SO₄). δ 8.47 (br s, 2H, pyrazole C-H), 8.35 (br d, 2H, aryl C-H), 7.88 (br t, 1 H, aryl C-H) ppm. ¹⁹F-NMR (376.5 MHz, neat D₂SO₄). δ -113.78 ppm (s, 1F, aryl C-F).

### Crystalline Porous MOF Ni₈-tet-F

1,4-bis[1-(4-pyrazolyl-)-2-fluorophenyl]tetrazine (BPAT) reacts with Ni(OAc)₂(H₂O)₄ in a 6:1 mixture v/v of DMF to DI water at 150 °C for 5 hours to yield crude (Ni₈(OH)₅(H₂O)₂(BPAT)₅ ). The solids are collected on a frit, washed with DMF (2x50 mL), methanol (2x50 mL), and acetone (2x 50 mL), and activated under high vacuum at 110°C for 12 hours to yield activated Ni₈-tet-F.

### Synthesis of Crystalline Porous MOF Ni₈-tet-CF₃

### 4-pyrazolyl-3-trifluoromethylbenzonitrile:

A 1-liter 3-neck RBF was charged with 4-bromo-3-trifluoromethylbenzonitrile (15.0 g, 60.0 mmol), 1-Boc-pyrazole-4-boronic acid pinacol ester (25.0 g, 85.0 mmol), PdCl₂(PPh₃)₂ (2.88 g, 4.10 mmol), NaNO₂ (17.25 g, 162.8 mmol) and a 4" stir bar. The flask was affixed with two stoppers and a condenser, and the system was evacuated and backfilled with N₂ three times.

Under N₂, DMF (375 mL, dry & deaerated) was added via cannula, followed by H₂O (75 mL, vigorously sparged with N₂) via syringe. The contents were stirred while solvent was being transferred. The vessel was then heated to reflux and vigorously stirred overnight (all contents except Na₂CO₃ dissolve to form an orange solution upon reaching temperature).

The flask was then opened to air and quenched by adding H₂O (420 mL) and EtOAc (750 mL) together in one portion. The contents were partitioned in a sep funnel and the organic phase was collected. The aqueous phase was washed with EtOAc (3 x 200 mL). The combined organic extractions were washed with brine (400 mL), dried over MgSO4, then filtered and the volatiles removed to yield yellow solids.

The solid residue was purified by flash chromatography on a 400 gram silica gel column with the following conditions: DCM (400 mL), then 50:50 DCM/EtOAc (600 mL), then EtOAc (400 mL). Fractions were checked by TLC, and all fractions containing the product were combined and solvent removed. The yellow solid residue was then taken up in hexanes and transferred to a frit, washed with hexanes (3 x 100 mL), and dried under vacuum overnight to yield pure 4-pyrazolyl-3-trifluoromethylbenzonitrile (10.5 g yellow solids, 44.3 mmol, 74% yield). ¹H-NMR (400 MHz, DMSO-d6). δ 13.30 (br s, 2H, pyrazole N-H), 8.33 (d, 2H, aryl C-H), 8.15 (dd, 2H, aryl C-H), 8.08 (s, 2 H, pyrazole C-H), 7.80 (d, 2H, aryl C-H), 7.76 (s, 2H, pyrazole C-H) ppm. ¹⁹F-NMR (376.5 MHz, DMSO-d6). δ -57.86 ppm (s, 3F, aryl CF₃).

### 1,4-bis[1-(4-pyrazolyl-)-2-(trifluoromethyl)phenyl]tetrazine (BPAT-CF₃)

To a 200 mL Schlenk flask were added 4-pyrazolyl-3-trifluoromethylbenzonitrile (10.5 g, 44.3 mmol), N-acetyl-L-cysteine (7.34 g, 45.2 mmol), and a 2" stir bar. The flask was stopped with a rubber septum, then evacuated and backfilled with nitrogen three times.

Under nitrogen, MeOH (90 mL, dry & deaerated) was added to the flask via cannula and contents stirred until fully dissolved, making a neon orange solution. Then, hydrazine monohydrate (9.0 mL, 190 mmol) was added slowly via syringe while stirring. After addition of hydrazine was complete, the reaction was stirred under N₂ at room temperature for 72 h, during which yellow solids precipitate from solution.

The flask was then opened to air and the orange solids collected on a frit. The crude dihydrotetrazine was washed with MeOH (2 x 20 mL), then dried for ca. 1 h under vacuum. The dried solids were then transferred to a 2 L round-bottom flask and sonicated in H₂O (1 L) until a fine suspension is formed.

At room temperature, with rapid stirring, NaNO₂ (17.7 g, 257 mmol) was added, followed by slow addition of glacial AcOH (500 mL). Upon addition of the glacial AcOH the color of the suspension changes from orange to pink over about 20 seconds (s). The suspension was stirred for about 5 min, then an additional portion of glacial AcOH (100 mL) was added and the suspension was stirred for 1 h.

The resulting pink solids were collected on a frit, washed with copious H₂O (4 x 200 mL), and dried on the frit over two days to yield pure BPAT-CF₃ (5.27 g fuschia solids, 10.5 mmol, 47.4% yield). ¹H-NMR (400 MHz, DMSO-d6): δ 13.28 (br s, 2H, pyrazole N-H), 8.88 (d, 2H, aryl C-H), 8.79 (dd, 2H, aryl C-H), 8.12 (br s, 2 H, pyrazole C-H), 7.94 (d, 2H, aryl C-H), 7.82 (br s, 2 H, pyrazole C-H) ppm. ¹⁹F-NMR (376.5 MHz, DMSO-d6). δ -57.73 (s, 3F, CF₃) ppm.

### Crystalline Porous MOF Ni₈-tet-CF₃

Stock solutions of BPAT-CF₃ (1.40 g, 2.79 mmol) in DMF (112 mL) and of Ni(OAc)₂ (0.972 g, 3.91 mmol) in H₂O (28 mL) were prepared in separate flasks, sonicating each flask until all solids are dissolved. The stock solutions were then combined in a 250 mL Erlenmeyer flask and swirled to thoroughly mix, resulting in the immediate formation of an orange-brown suspension.

The following steps were carried out in four portions: 35 mL of the BPAT-CF3 + Ni(OAc)₂ suspension was transferred to a microwave vessel (CEM Discover with temperature probe), which was capped and microwaved to maintain 155 °C for 5 min with rapid stirring. The microwave vessel was allowed to cool to room temperature, and then the solid contents were collected by centrifugation and washed with DMF (3 x 40 mL), H₂O (3 x 40 mL), and acetone (2 x 40 mL), agitating the solid cake between washes and soaking in each solvent for at least an hour.

After each portion was washed, the four portions were combined and activated (dried of residual solvent by heating under vacuum) overnight at 145 °C to yield Ni₈-tet-CF₃ (1.28 g terracotta solids, 0.357 mmol, 77% yield). BET Surface Area: 3620 ± 110 m²/g.

### Synthesis of Crystalline Porous MOF UiO-68-tet (not according to the invention)

### 4,4'-(1,2,4,5-tetrazine-3,6-diyl)bis(2-fluorobenzoic acid) (BBAT)

A 100 mL sidearm Schlenk flask was charged with 4-cyano-2-fluorobenzoic acid (1.8 g, 10.9 mmol), EtOH (40 mL) and a 1" stir bar, and the contents of the flask stirred until acid dissolved. A 20 cm condenser was affixed to the flask and the system purged vigorously with N₂ for 6 min. Under positive pressure of N₂, hydrazine monohydrate (5.4 mL, 110 mmol) was added pipettewise. Upon addition the homogeneous solution immediately precipitated a white solid. The flask was purged an additional 2 min with N₂, and then the reaction heated in a 90 °C oil bath for 19.5 h with a N₂ feed affixed to the top of the condenser. After heating, the flask was removed from the oil bath and cooled for 20 min under N₂. The flask was then opened to air and the solids collected on a medium frit.

The crude yellow product was filtered to dampness, then washed with DI water (30 mL, then 10 mL), then EtOH (2 x 10 mL, then 20 mL), and then filtered to dryness. The solid cake was agitated between each wash and the washes were filtered to dampness. Solids were transferred to a plastic weigh dish and dried under room temperature air for 3 h to obtain the dihydrotetrazine as crusty tan solids.

Next, a 100 mL round bottom flask charged with glacial AcOH (16 mL), H₂O (10 mL) and a 1" stir bar was cooled in an ice water bath for 10 min. While stirring, the crude dihydrotetrazine was transferred to the flask to form a tan suspension. An addition funnel was affixed to the flask and filled with a solution of NaNO₂ (1.95 g) dissolved in H₂O (14 mL). Still stirring, the NaNO₂ solution was added dropwise at a rate of 1 drop every 2 s. During the addition, the suspension changed in color from yellow/tan to pink. After the addition was complete, the addition funnel was removed and the suspension left to stir for another 5 min, still over ice. The flask was then removed from the ice bath and sonicated for 30 s, then stirred at room temperature for 45 min.

The resulting pink solids were recovered by centrifugation and washed with H₂O (3 x 10 mL), then EtOH (2 x 20 mL), agitating the solids between each wash. The solids were finally dried in vacuo to yield BBAT (524 mg fuschia solids, 1.46 mmol, 27% yield). ¹H-NMR (400 MHz, DMSO + drop NEt₃). δ 7.80 (1H, t), 8.13 (1H, dd), 8.28 (1H, dd) ppm. ¹⁹F-NMR (376.5 MHz, DMSO-d6 + drop NEt₃). δ -113.13 ppm (s, 1F, aryl C-F).

### Crystalline Porous MOF-UiO-68-tet

A 500 mL glass bottle was charged with benzoic acid (3.93 g, 32.2 mmol) and DMF (60 mL). ZrCl₄ (170 mg, 0.73 mmol) was added and the mixture sonicated until homogeneous, then the bottle was placed in a 110 °C oven for 20 min. The bottle was then removed from the oven and allowed to cool for 5 min. BBAT (260 mg, 0.73 mmol) was then added to glass bottle and the mixture sonicated for 5 min. The bottle was returned to the 110 °C oven for 3 days, during which pink crystals form. The bottle was then removed from the oven and allowed to cool to room temperature. The pink crystals were then collected on a glass frit and washed with DMF (3 x 20 mL), then Et₂O (2 x 20 mL). The solids were dried on the frit, then transferred to a glass vial and activated for 2 days at 55 °C under vacuum to yield UiO-68-tet (105.62 mg hot pink microcrystalline solids, 0.0373 mmol, 30.7% yield). BET surface area: 2,048 ± 54 m²/g.

### Synthesis of Crystalline Porous MOF-Mg-IRMOF-74-III-tet (not according to the invention)

### 5-cyanosalicylic acid

A 500 mL round-bottom flask was charged with 5-formylsalicylic acid (5.00 g, 30.1 mmol), hydroxylamine HCl (6.27 g, 105 mmol), lithium formate (7.35 g, 90.3 mmol), formic acid (59.5 mL), and a 2" stir bar. The flask was capped with a rubber septum and the headspace purged with N₂ for 20 min. The purge needle was removed, then the flask transferred to a preheated 70 °C oil bath and stirred overnight. After heating, the reaction was allowed to cool to room temperature, and two thirds of the solvent was removed by heating to 50 °C under vacuum. Upon concentration, the homogeneous solution precipitates white solids. The concentrated reaction mixture was allowed to sit at room temperature for 1 h, then the solids were collected on a frit and washed with formic acid (5 mL) followed by ice cold water (3 x 5 mL). The product was dried under vacuum overnight to yield 5-cyanosalicylic acid (4.46 g white solids, 27.3 mmol, 91%). ¹H-NMR (400 MHz, DMSO-d6). δ 8.18 (1H, d, aryl C-H), 7.91 (1H, dd, aryl C-H), 7.12 (1H, d, aryl C-H) ppm.

### 5,5'-(1,2,4,5-tetrazine-3,6-diyl)bis(2-hydroxybenzoic acid) (BHBAT)

A 100 mL Schlenk flask was charged with 5-cyanosalicylic acid (4.46 g, 27.3 mmol), N-acetyl-L-cysteine (4.50 g, 27.6 mmol), and a ½" stir bar. The flask was evacuated and backfilled with N₂ twice, then MeOH (50 mL, dry & deaerated) was transferred to the Schlenk flask via cannula to form a white suspension. Under positive pressure of N₂, and with rapid stirring, hydrazine hydrate (4.5 mL, 91.8 mmol) was added quickly to the mixture. The vessel was sealed under N₂ and heated to 50 °C for 3 days, during which time the suspension changes in color to yellow-orange.

After heating, the suspended solids were collected on a glass frit. The solids were washed with ethanol (5 mL) followed by DI water (5 mL). The solids were then collected and resuspended in DI water (50 mL). The yellow suspension was sonicated for 5 minutes, then titrated to pH = 1 using 6 M sulfuric acid. The suspension was sonicated again for one minute, then filtered on a glass frit. The solids were collected and resuspended in ethanol (100 mL) and sonicated for 5 minutes. While stirring, benzoquinone was added to the ethanol suspension in 250 mg portions, turning the solution a deep red color. The suspension was stirred for one hour, then filtered, washed with copious ethanol, and dried on the frit. The solids were resuspended in THF (15 mL), sonicated, and filtered to remove any residual benzoquinone. The red solids were fully dried on the frit to yield BHBAT (0.9986 g red solids, 2.82 mmol, 20% yield). ¹H-NMR (400 MHz, DMSO). δ 8.97 (d, 1H, aryl C-H), 8.62 (dd, 1H, aryl C-H), 7.26 (d, 1H, aryl C-H) ppm.

### Crystalline Porous MOF-Mg-IRMOF-74-III-tet

A 20 mL scintillation vial was charged with Mg(NO₃)₂[H₂O]₆ (36.7 mg, 0.143 mmol), BHBAT (25 mg, 0.071 mmol), DMF (3 mL), and EtOH (3 mL). The suspension was sonicated for 10 min, then the vial tightly capped and transferred to a 130 °C oven for 2 days. The vial was then removed from the oven and allowed to cool to room temperature. The solids were collected by centrifugation and washed with MeOH (2 x 10 mL), soaking in each wash for at least 1 h. The washed MOF was dried at a temperature of 100 °C in vacuo to yield Mg-IRMOF-74-III-tet.

### Grafting of Phosphorus Ligands on Crystalline Porous MOF to Form Heterogenous Catalyst Precursor

### General Procedure for Grafting Phosphorus Ligands with Crystalline Porous MOF to Form Heterogenous Catalyst Precursor

Under a N₂ atmosphere, activated crystalline porous tetrazine MOF (50 mg) is added to a scintillation vial. A stock solution of the phosphorus ligand (*e.g.,* Nor1, Norphos, allyldiphenylphosphine, 0.1-1 equiv vs. MOF tetrazine) in DCM or DMF (2 mL, dry & deaerated) is prepared separately and added to the vial containing the MOF, ensuring the MOF is fully immersed in solvent. The vial is then tightly sealed and heated to 60 °C under an inert atmosphere (N₂ gas) for 48 hours, then washed with fresh solvent (DCM or DMF, 3 x 5 mL), soaking in each wash for at least 1 h. The material is then activated by heating under high vacuum overnight to yield the heterogenous catalyst precursor.

### Nor1@Ni₈-tet-F

Following the general procedure, using 52 mg of activated Ni₈-tet-F and 5.2 mg of Nor1. Activation temperature: 80 °C.

### Nor1@UiO-68-tet (not according to the invention)

Following the general procedure, using 75 mg of activated UiO-68-tet and 7.5 mg of Nor1. Activation temperature: 50 °C.

### Norphos@Ni₈-tet-F

Following the general procedure, using 52 mg of activated Ni₈-tet-F and 5.2 mg of Norphos. Activation temperature: 80 °C.

### Norphos@Ni₈-tet-CF₃

Following the general procedure, using 50 mg of activated Ni₈-tet-CF₃ and 5.0 mg of Norphos. Activation temperature: 80 °C.

### Allyldiphenylphosphine + UiO-68-tet (not according to the invention)

Following the general procedure, using 75 mg of activated UiO-68-tet and 7.5 mg of allyldiphenylphosphine. Activation temperature: 50 °C.

### Enamine-PPh2 + ViO-68-tet (not according to the invention)

In an N₂ glovebox, UiO-68-tet (75 mg) was suspended in anhydrous DCM (5 mL). This solution was let stand for 5 minutes before 3-(diphenylphosphaneyl)cyclopentan-1-one (7.5 mg) was added to the suspension. Anhydrous pyrrolidine (about 10 eq to ketone, 28 µL, 3.1 mmol) was added to the suspension which was then gently shaken overnight. The next day the color had change from bright fuchsia to a rustier red. The solids were collected on a frit, washed with anhydrous DCM (3 x 10 mL), hexane (2 x 10 mL) and dried under vacuum. After drying with normal vacuum, the MOF was transferred to activation glassware and activated under high vacuum at 50 °C for 12-20 hours (hr).

### Enamine-PPh2 + Ni₈-tet-F

In an N₂ glovebox, Ni₈-tet-F (75 mg) was suspended in anhydrous DCM (5 mL). This solution was let stand for 5 minutes before 3-(diphenylphosphaneyl)cyclopentan-1-one (7.5 mg) was added to the suspension. Anhydrous pyrrolidine (about 18 eq to ketone, 50 uL, 5.5 mmol) was added to the suspension which was then gently shaken overnight. The next day the color had change from tan to a darker tan-brown. The solids were collected on a frit, washed with anhydrous DCM (3 x 10 mL), hexane (2 x 10 mL) and dried under vacuum. After drying with normal vacuum, the MOF was transferred to activation glassware and activated under high vacuum at 50 °C for 12-20 hr.

### Rh(acac)(Norphos)@Ni₈-tet-CF₃, direct grafting of Rh(acac)(Norphos)

Rh(Norphos)(acac)@Ni₈-tet-CF₃ was prepared using a molecular Rh(Norphos)(acac) complex following the general procedure for grafting using 100 mg of Ni₈-tet-CF₃ and 10 mg of Rh(acac)(Norphos).

### Norbornene Treatment of Heterogenous Catalyst Precursor

Heterogenous catalyst precursors, formed following the general procedure for grafting phosphorus ligands with the crystalline porous MOF, were treated with excess norbornene to avoid side reactions or decomposition of the remaining tetrazine groups. The treatment is performed by the following procedure: Under an N₂ atmosphere, 50 mg of the heterogenous catalyst precursor is immersed in a stock solution of norbornene (about 1 M in 1 mL DCM) and heated overnight at 60 °C. The heterogenous catalyst precursor is then washed with fresh DCM (1 x 5 mL), then fresh hexanes (2 x 5 mL), soaking in each wash for at least 1 h. The heterogenous catalyst precursor is then activated by heating under high vacuum, as described above, overnight to yield the heterogenous catalyst precursor.

### Metallation of the Heterogenous Catalyst Precursor to Form the Heterogeneous Catalyst Composition

### General procedure for metallation of the Heterogenous Catalyst Precursor to Form the Heterogeneous Catalyst Composition

Under a N₂ atmosphere, the heterogeneous catalyst precursor (50 mg) is suspended in a minimum of DCM, Et₂O, or THF (2 mL, dry & deaerated) in a 20 mL scintillation vial. The Group VIII transition metal catalyst precursor compound (0.9 equiv vs. grafted phosphorus) is then added as a stock solution (about 0.02 M in DCM) and the vial swirled to combine. The suspension is allowed to sit an inert atmosphere (N₂ gas) for 18 hours, then collected on a frit, washed with DCM (3 x 4 mL), and dried under vacuum to yield the heterogeneous catalyst composition.

Example procedure for metalation of Norphos@Ni8-tet-CF3: Under a N₂ atmosphere, Norphos@Ni8-tet-CF3 (325 mg, 1 eq Norphos per MOF tetrazine linker) is immersed in a solution of Rh(acac)(CO)₂ (6.2 mg, 0.024 mmol) in THF (2 mL, dry and degassed) in a 20 mL scintillation vial. The suspension is allowed to sit under an inert atmosphere (N₂ gas) overnight, then collected on a frit, washed with THF (3 x 4 mL), and dried under vacuum to yield Rh(acac)(Norphos)@Ni8-tet-CF3 with high ligand concentration.

### Hydroformylation Catalysis

### General Procedure, Hydroformylation of 1-octene by Rh(acac)(Norphos)@Ni₈-tet-CF₃

In a N₂ glovebox, a 25 mL stainless steel reactor vessel is charged with Rh(acac)(Norphos)@Ni₈-tet-CF₃ (10 mg, 0.003 mmol), 1-octene (0.5 mL, 3.18 mmol), and a 1/2" stir bar. The vessel is closed, removed from the glovebox, affixed to a gas manifold, and inserted into a heating mantle. With the vessel still closed, the manifold is evacuated & backfilled with N₂ three times. The vessel is then opened to the manifold and pressurized with syngas (1:1 H₂/CO) to 400 psig. The reactor is then closed & the manifold vented. The heating mantle is set to 100 °C and stirred at 400 rpm, and the reaction is then left for 4 h. The reactor is then removed from the heat, immersed in an ice bath for 5 min, then carefully vented. 1-chlorobutane (0.04 mL) is added as an internal standard, then the reaction mixture is diluted 10-fold with bench toluene and filtered.

Reaction products were analyzed by GC-MS, using a calibration curve prepared from authentic standards of 1-octene, 1-nonanal, and 1-chlorobutane (internal standard).

### General Procedure, Hydroformylation of 1-pentene by Rh(acac)(Norphos)@Ni₈-tet-CF₃

In a N₂ glovebox, a 25 mL stainless steel reactor vessel is charged with Rh(acac)(Norphos)@Ni₈-tet-CF₃ (10 mg, 0.003 mmol), 1-pentene (0.25 mL, 2.28 mmol), toluene (1.0 mL, dry and degassed), and a 1/2" stir bar. The vessel is closed, removed from the glovebox, affixed to a gas manifold, and inserted into a heating mantle. With the vessel still closed, the manifold is evacuated & backfilled with N₂ three times. The vessel is then opened to the manifold and pressurized with syngas (1:1 H₂/CO) to 400 psig. The reactor is then closed & the manifold vented. The heating mantle is set to 100 °C and stirred at 400 rpm, and the reaction is then left for 4 h. The reactor is then removed from the heat, immersed in an ice bath for 5 min, then carefully vented. 1-chlorobutane (0.04 mL) is added as an internal standard, then the reaction mixture is diluted 10-fold with bench toluene and filtered.

Reaction products were analyzed by GC-MS, using a calibration curve prepared from authentic standards of 1-octene, 1-nonanal, and 1-chlorobutane (internal standard).

### Recycling procedure, hydroformylation of 1-octene by Rh(acac)Norphos@Ni₈-tet-CF₃.

After hydroformylation catalysis, the reactor (sealed and under pressure of synthesis gas) is removed from heat and allowed to cool. The reactor is then vented to *ca.* 100 psig and connected to a N₂ manifold with gas bubbler. The remaining syngas is vented through the bubbler and the reactor held under a flow of N₂ for 30 min with stirring. The purged reactor is then brought into a N₂ glovebox, where the reactor is opened, the product mixture collected by decanting + filtering, and the remaining catalyst solids taken up in wash solvent (toluene or Et₂O, 5 mL) and transferred to a 20 mL scintillation vial.

The catalyst is triturated with the wash solvent 3x5 mL, then immersed in 5 mL of wash solvent & soaked 1 h. The wash is then replaced with fresh solvent and the catalyst soaked again for 1 h. The catalyst is then triturated 3x5 mL with hexanes, then soaked once in 5 mL of fresh hexanes for 1 h, and then finally the solvent is decanted and the solids dried in vacuo to yield the recycled catalyst.

### General procedure for hydroformylation of vinyltrimethoxysilane by Rh(acac)Norphos@Nis-tet-CF₃

In a N₂ glovebox, a stainless steel Parr reactor is charged with Rh(acac)(Norphos)@Ni8-tet-CF3 (20 mg, 0.006 mmol), vinyltrimethoxysilane (5 mL, 32.7 mmol), and a 1" stir bar. The vessel is sealed, removed from the glovebox, and affixed to a gas manifold which was previously evacuated & backfilled with N₂ three times. The Parr reactor is then opened to the manifold and pressurized with syngas (1:1 H₂/CO) to the desired pressure. The reactor is heated to the reaction temperature and stirred at 300 rpm for the duration of the reaction, typically 4 h. The reactor is then sealed, removed from the heating block, and immersed in an ice bath for 5 min until the reactor reaches room temperature, then the reactor is carefully vented. 1,4-dioxane (0.05 mL) is added as an internal standard for NMR analysis, then the reaction mixture is decanted from the solid catalyst, filtered, and submitted for ¹H-NMR.

### Quantification of vinyltrimethoxysilane hydroformylation products by NMR

Reaction products were analyzed by NMR (0.01 mL product mixture in CDCl₃). The starting material was quantified by integrating the vinyl protons (6.2-5.8 ppm, m, 3H), the n aldehyde product quantified by the aldehyde C(O)-H proton (9.77 ppm, t, 1H), and the iso aldehyde product quantified by the aldehyde C(O)-H proton (9.74 ppm, d, 1H). The mmol of starting material and products were calculated by referencing to the internal standard 1,4-dioxane (3.70 ppm, s, 8H). NMR data was collected on a 500 MHz spectrometer using a 10 second relaxation delay between scans.

### Recycling experiment, hydroformylation of vinyltrimethoxysilane by Rh(acac)Norphos@Ni₈-tet-CF₃

Following the general procedure, 30 mg of MOF catalyst and 7.5 mL of vinyltrimethoxysilane were allowed to react in a Parr reactor vessel for 3.5 h at 600 psig of synthesis gas and 100 °C. The reactor was then vented to a pressure of ~100 psig, then affixed to a N₂ manifold and the headspace purged with N₂ for 20 min. The reactor was then sealed under the N₂ atmosphere and returned to a N₂ glovebox, where the reactor was opened, the reaction mixture decanted, and the catalyst transferred to a 20 mL scintillation vial. The used MOF catalyst was triturated 3 x 5 mL with diethyl ether, then soaked in 10 mL of diethyl ether for ~1 h. The diethyl ether soak solution was then decanted and the solids triturated 2 x 5 mL with hexanes, then soaked in 10 mL of hexanes for ~30 min. The hexanes soak solution was then decanted and the solids dried under vacuum, to be used in a subsequent catalytic reaction.

**Table 1. Hydroformylation of 1-pentene and 1-octene by Rh(acac)(Norphos)@Ni8-tet-CF3**

| entry | catalyst | Substrate | S/C*^{a}* | % CHO.*^{b}* | %C=C isomer^{c} | n/iso*^{d}* | TON*^{d}* | TOF (h⁻¹)^{e} |
|---|---|---|---|---|---|---|---|---|
| 1 | Rh(acac)(Norphos) @Ni8-tet-CF3 | 1-octene | 29000 | 8 | 24 | 4.0 | 1900 | 480 |
| 2 | Rh(acac)(Norphos) | 1-octene | 2900 | 28 | 66 | 2.9 | 530 | 130 |
| 3 | Rh(acac)Norphos (molecular) | 1-octene | 2650 | 31 | 10 | 2.8 | 630 | 160 |
| 4 | Rh(acac)(Norphos) @Ni8-tet-CF3' | 1-octene | 19000 | 12 | 47 | 3.5 | 1900 | 475 |
| 5 | Rh(acac)(Norphos) @Ni8-tet-CF3*ⁱⁱ* | 1-octene | 13000 0 | 7 | 32 | 3.1 | 8100 | 2000 |
| 6 | Rh(acac)(Norphos) @Ni8-tet-CF3^{*ii*i} | 1-octene | 16000 0 | 9 | 79 | 2.9 | 1190 0 | 2970 |
| 7 | Rh(BF4)(Norphos) @Ni8-tet-CF3 | 1-octene | 24000 | 23 | 52 | 0.7 | 4900 | 1230 |
| 8 | Rh(acac)(Norphos) @Ni8-tet-CF3 | 1-pentene | 16000 | 3 | 29 | -* | 390 | 100 |
| 9 | Rh(BF4)(Norphos) @Ni8-tet-CF3 | 1-pentene | 25000 | 7 | 43 | -* | 1100 | 290 |
| 10 | Rh(BF4)(Norphos) @Ni8-tet-CF3*^{iv}* | 1-pentene | 17000 | 0.6 | 12 | -* | 50 | 10 |

| | | | | | | | | |
|---|---|---|---|---|---|---|---|---|
| ^{a}S/C = mmol_{substrate}/mmol_{Rh}. *^{b}%CHO* = mol_{aldehyde}/molₜₒₜₐₗ. ^{c}%C=C isomer = extent of double bond isomerization without hydroformylation molᵢₛₒₘₑᵣ/molₜₒₜₐₗ. *^{d}*Turnover number (TON) = mmol_{aldehydes}/mmol_{Rh}. ^{e}Turnover frequency (TOF) = TON / time_{reaction}. *Branched product not detected for hydroformylation of 1-pentene. *ⁱ*Toluene volume increased from 0.5 mL to 1.0 mL *ⁱⁱ*Reaction run in neat 1-octene (1.0 mL, to keep catalyst immersed) *ⁱⁱⁱ*Reaction scaled up 10-fold *^{iv}*Syngas pressure lowered to 300 psig | | | | | | | | |

**Table 2. Hydroformylation of Vinyltrimethoxysilane - Catalyst Details**

| **Entry** | **Catalyst** | **Nor/Tz** | **Rh/Nor** | **umol Rh/ mg MOF Notes** | |
|---|---|---|---|---|---|
| **1** | Rh(acac)(Norphos)@Ni8-tet-CF3 | 0.083 | 1.0 | 0.0140 | |
| **2** | Rh(acac)(Norphos) @ Ni8-tet-CF3 | 0.083 | 1.0 | 0.0140 | |
| **3** | Rh(acac)(Norphos) @ Ni8-tet-CF3 | 0.083 | 1.0 | 0.0082 | Recycled from entry 2 |
| **4** | Rh(acac)(Norphos)@Ni8-tet-CF3 | 0.083 | 1.0 | 0.0140 | |
| **5** | Rh(acac)(Norphos) @ Ni8-tet-CF3 | 1.0 | 0.10 | 0.012 | |
| **6** | Rh(acac)(Norphos) @ Ni8-tet-CF3 | 1.0 | 0.10 | 0.012 | |
| **7** | Rh(acac)(Norphos) | n.a. | 1.0 | n.a. | isolated Rh(Norphos) complex |
| **8** | Rh(acac) + Norphos | n.a. | 0.33 | n.a. | Rh(acac)(CO)2 + Norphos combined in situ |

**Table 3. Hydroformylation of Vinyltrimethoxysilane - Reaction Parameters**

| **Entry** | **Substrate Loading** | | | **Catalyst Loading** | | **s/c** | **Temp** | **Syngas** | **Time** |
|---|---|---|---|---|---|---|---|---|---|
| | *mL* | *g* | *mmol* | *mg MOF* | *umol Rh* | | °*C* | *psig* | *h* |
| **1** | 5 | 4.8 | 32.7 | 20.2 | 0.28 | 116000 | 100 | 400 | 4 |
| **2** | 5 | 4.8 | 32.7 | 20.6 | 0.29 | 113000 | 100 | 600 | 4 |
| **3** | 5 | 4.8 | 32.7 | 20.1 | 0.16 | 199000 | 100 | 600 | 4 |
| **4** | 5 | 4.8 | 32.7 | 20.3 | 0.28 | 115000 | 80 | 600 | 4 |
| **5** | 5 | 4.8 | 32.7 | 59.7 | 0.74 | 44000 | 100 | 600 | 4 |
| **6** | 5 | 4.8 | 32.7 | 20.1 | 0.25 | 131000 | 100 | 600 | 4 |
| **7** | 15 | 14.5 | 98.10 | - | 2.45 | 40000 | 100 | 600 | 4 |
| **8** | 4 | 3.9 | 26.12 | - | 0.10 | 272000 | 100 | 600 | 4 |

**Table 4. Hydroformylation of Vinyltrimethoxysilane: Reaction Data**

| **Entry** | **alkene loading** | **n yield** | **iso yield** | **total aldehyde** | **alkene** | **Conv.** | **n/iso** | **TON** | **TOF** | | **mass balance** |
|---|---|---|---|---|---|---|---|---|---|---|---|
| | *mmol* | *mmol* | *mmol* | *mmol* | *mmol* | | | | *s*⁻¹ | *h⁻¹* | *(%)* |
| **1** | 32.7 | 3.44 | 2.36 | 5.80 | 24.5 | 19.1% | 1.5 | 20500 | 1.42 | 5125 | 93% |
| **2** | 32.7 | 2.48 | 1.27 | 3.75 | 28.9 | 11.5% | 1.9 | 13000 | 0.90 | 3250 | 100% |
| **3** | 32.7 | 2.11 | 1.17 | 3.28 | 29.9 | 9.9% | 1.8 | 20000 | 1.39 | 5000 | 101% |
| **4** | 32.7 | 0.32 | 0.19 | 0.51 | 31.3 | 1.6% | 1.7 | 1800 | 0.13 | 450 | 97% |
| **5** | 32.7 | 0.86 | 0.06 | 0.92 | 28.8 | 3.0% | 13.4 | 1250 | 0.09 | 310 | 91% |
| **6** | 32.7 | 0.78 | 0.03 | 0.81 | 29.0 | 2.6% | 26.5 | 3250 | 0.23 | 810 | 91% |
| **7** | 98.1 | 3.13 | 3.46 | 6.58 | 89.9 | 6.4% | 0.9 | 2700 | 0.19 | 675 | 98% |
| **8** | 26.1 | 0.74 | 0.07 | 0.81 | 21.2 | 3.5% | 10.3 | 8400 | 0.58 | 2100 | 84% |

### Comments on Results of Tables 1-4

Hydroformylation of 1-octene and 1-pentene (Table 1). Rh(acac)Norphos@Ni8-tet-CF3 closely reproduces activity and selectivity of molecular catalyst for hydroformylation of 1-octene (entry 3). Rh(acac)Norphos@Ni8-tet-CF3 achieves the highest selectivity for linear aldehydes of any MOF catalyst reported to-date (n/iso = 4.0, entry 1), while maintaining comparable or superior activity in terms of TON and TOF. Choice of Rh precursor has marked effect on selectivity: MOF catalysts prepared from Rh(acac)(CO)₂ favored linear products (n/iso ≥ 2.8, entries 1, 2, 4-6) but MOF catalysts prepared from Rh(1,5-COD)₂(BF4) favored branched products (n/iso = 0.7, entry 7). Choice of Rh precursor has marked effect on activity: MOF catalyst prepared from Rh(1,5-COD)₂(BF4) was nearly 3 times more active (TON = 4900) than a MOF catalyst prepared from Rh(acac)(CO)2 (TON = 1900) under similar conditions (entries 2 & 7).

Hydroformylation of vinyltrimethoxysilane (Tables 2-4). MOF catalysts (entries 1-6) reproduced both the activity and selectivity of the molecular analogs (entries 7 & 8). MOF catalysts prepared with a high phosphorus ligand concentration (Nor/Tz = 1, Rh/Nor = 0.1) displayed exceptional selectivity for the linear aldehyde, reaching n/iso = 26.5 (entries 5 & 6). MOF catalysts prepared with a low phosphorus ligand concentration (Nor/Tz = 0.083, Rh/Nor = 1) did not exceed n/iso = 2 (entries 1-4). No other MOF catalyst has achieved selectivity for linear aldehydes greater than n/iso = 3.2 for any alkene substrate. High phosphorus ligand concentration resulted in a decrease of activity by TON. Increasing the synthesis gas pressure from 400 psig to 600 psig resulted in a slight increase of selectivity but a concomitant decrease in activity (entries 1 & 2). Decreasing reaction temperature from 100 ^{O}C to 80 ^{O}C resulted in a 10-fold reduction of catalyst activity without substantial effect on selectivity (entries 2 & 4).

## Claims

1. A crystalline porous metal-organic framework (MOF) comprising:
a plurality of non-catalytic metal ions; and
a plurality of linkers, wherein the plurality of non-catalytic metal ions coordinate with the plurality of linkers to form the crystalline porous MOF, wherein the plurality of linkers are formed from a compound of Formula I: 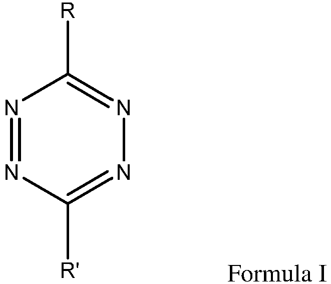
wherein each of R and R' is independently selected from: 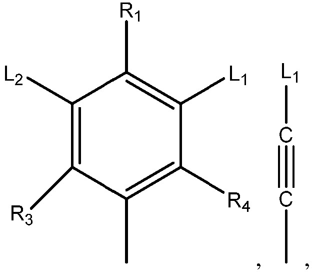 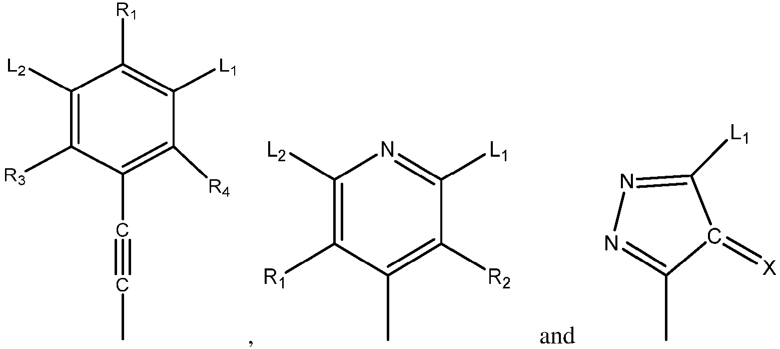
wherein each of R₁ and R₂ are independently H, C₁ to C₃ alkyl, -F, -Cl, -Br, - I, or -CF₃;
wherein each of R₃ and R₄ are independently H or C₁ to C₃ alkyl, wherein R₃ and R₄ can form a bridge structure;
X is selected from O and S;
L₁ and L₂ are each selected from the group: 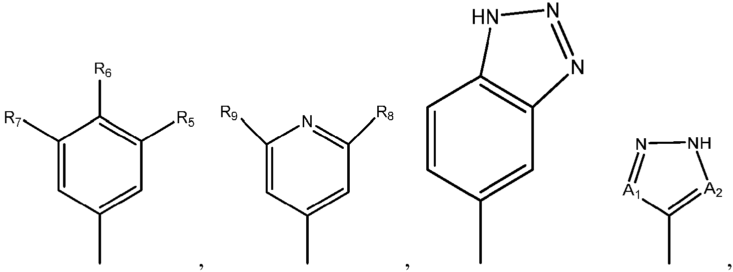 -COOH, -OH and -NH₂
wherein each of R₅, R₆ and R₇ are independently selected from -H -OR₁₀, -COOR₁₁, -NR₁₂, -F, -Cl, -Br, -I, or --CF₃, wherein R₁₀, R₁₁ and R₁₂ are each independently selected from -H or C₁ to C₃ alkyl;
wherein each R₈ and R₉ are independently selected from -H, -OH, -COOH, or -NH₂; and
A₁ and A₂ are either C or N;
wherein each of R and R' is: 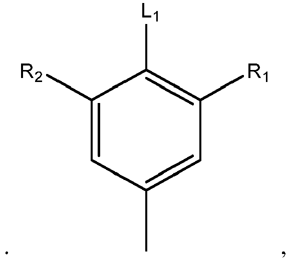 wherein L₁ is 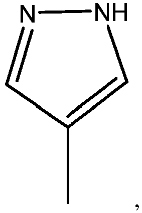 R₁ is -H, -F or -CF₃ and R₂ is -H.

2. The crystalline porous MOF of claim 1, wherein each of R and R' is: 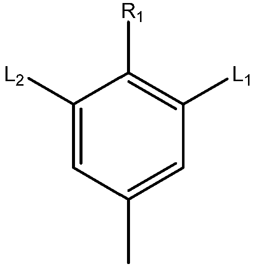 and L₁ and L₂ are

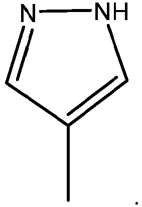

3. The crystalline porous MOF of claim 2, wherein R₁ is H.

4. The crystalline porous MOF of any one of claims 1- 3, wherein the plurality of non-catalytic metal ions are selected from the group consisting of nickel, magnesium, copper, cobalt, zirconium, iron, zinc, vanadium, aluminum and combinations thereof.

5. The crystalline porous MOF of any one of claims 1- 3, wherein the plurality of non-catalytic metal ions are nickel or magnesium.

6. A heterogeneous catalyst precursor, comprising:
a reaction product of the crystalline porous MOF of any one of claims 1- 5 and a phosphorous ligand of Formula II: 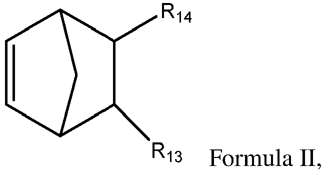
to form the heterogeneous catalyst precursor of Formula III: 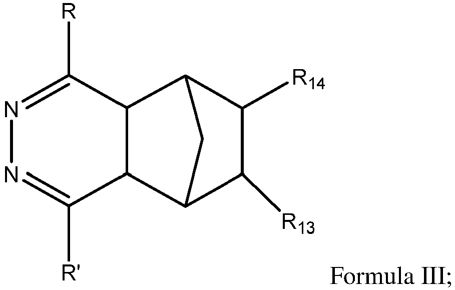 or
a phosphorous ligand of Formula IV: 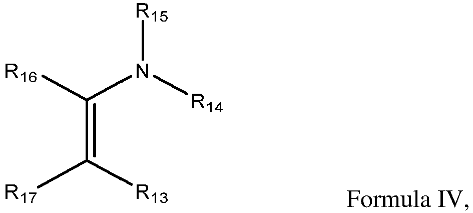
to form the heterogeneous catalyst precursor of Formula V: 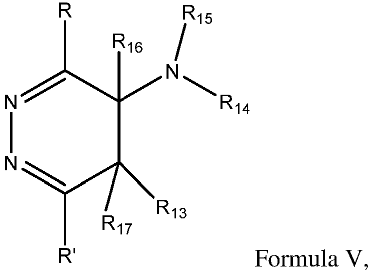 or 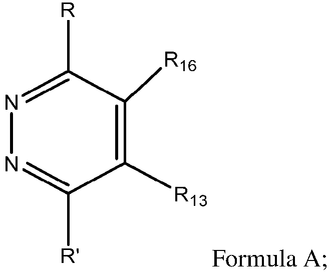 or
a phosphorous ligand of Formula VI: 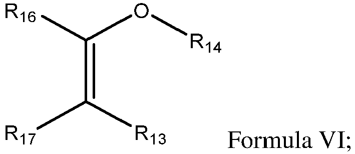
to form the heterogeneous catalyst precursor of Formula VII: 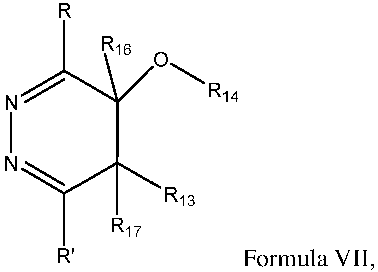 or 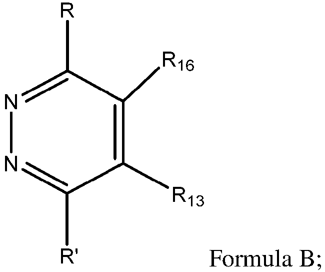
a phosphorous ligand of Formula VIII: 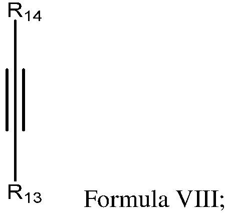 or 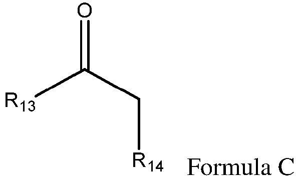 and an amine catalyst to form the heterogeneous catalyst precursor of Formula IX: 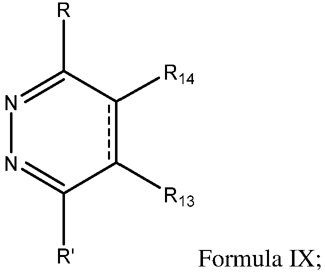 or
a phosphorous ligand of Formula D: 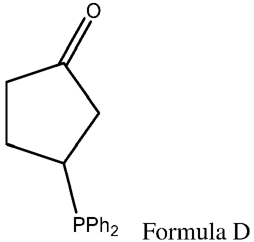
to form: 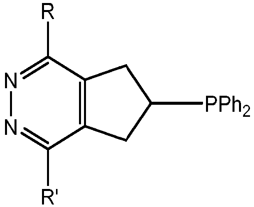
wherein R₁₆ and R₁₇ are each independently -H and C₁ to C₃ alkyl; and
wherein R₁₃ - R₁₅ are each independently selected from -H, C₁ to C₃₀ alkyl and at least one of: 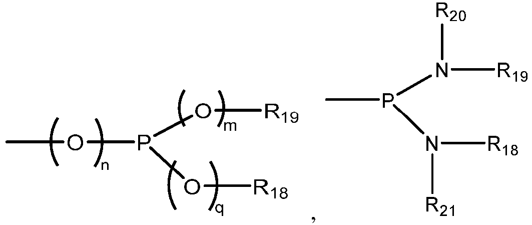 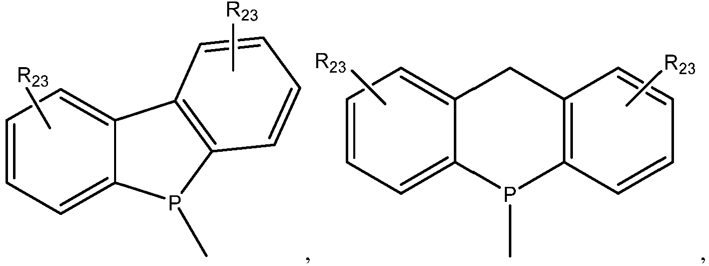 PPh₂ or 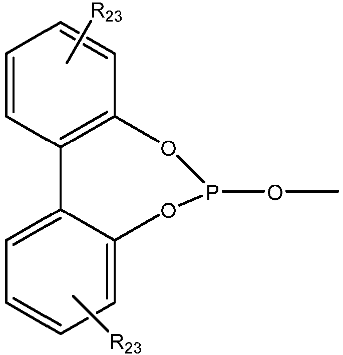
wherein n, m, and q are either 0 or 1, wherein (n,m,q) is either (1,0,0), (1,1,0), (0,0,1), (0,1,1) or (1,1,1);
wherein each of R₁₈ through R₂₁are selected from C₅-C₁₀ aryl, C₁-C₄ alkyl or -N(R₂₂)₂, wherein R₂₂ is selected from -H or C₁ to C₃ alkyl; and
wherein each of R₂₃ is selected from C₅-C₁₀ aryl, C₁-C₄ alkyl, -OR₂₂ or-N(R₂₂)₂, where R₂₂ is selected from -H or C₁ to C₃ alkyl.

7. The heterogeneous catalyst precursor of claim 6, wherein the heterogeneous catalyst precursor is of Formula III and R₁₃ and R₁₄ are each diphenylphosphine.

8. The heterogeneous catalyst precursor of claim 6, wherein R₁₃ is H and R₁₄ is PPh₂.

9. A heterogeneous catalyst composition for hydroformylation of olefins, comprising:
a reaction product of the heterogeneous catalyst precursor of any one of claims 6-8 with a Group VIII transition metal catalyst precursor compound.

10. The heterogeneous catalyst composition of claim 9, wherein the Group VIII transition metal catalyst precursor compound is of Formula X:
M_{w}(L¹)ₓ(L²)_{y}(L³)_{z} Formula X
wherein the M is selected from the group consisting of rhodium (Rh), cobalt (Co), iridium (Ir), ruthenium (Ru), iron (Fe), nickel (Ni), palladium (Pd), platinum (Pt), and osmium (Os);
L¹, L² and L³ are each independently selected from the group consisting of hydrogen, carbonyl (CO), cyclooctadiene, norbornene, chlorine, oxygen, boron, fluoride, bromide, iodide, nitrate, acetate, octanoate, 2-ethylhexanoate, triphenylphosphine (TPP), and acetylacetonate (AcAc);
w is an integer from 1 to 6; and
x, y and z are each independently an integer from 0 to 5 wherein the sum of x, y, and z is at least 1.0.

11. The heterogeneous catalyst of claim 10, wherein the transition metal catalyst of Formula X is selected from the group consisting of Ph(O₂C₅H₇)(CO)₂, Rh₂O₃, Rh₄(CO)₁₂, Rh₆(CO)₁₆, Rh(NO₃)₃, bis(norbornadiene)rhodium(I) tetrafluoroborate and bis(1,5-cyclooctadiene)rhodium(I) tetrafluoroborate.

12. The heterogeneous catalyst of claim 9, wherein the heterogeneous catalyst precursor is provided in a molar excess, based on phosphorous content, relative the Group VIII transition metal catalyst precursor compound.

13. A method of forming a heterogeneous catalyst, comprising:
reacting a compound of Formula I of any one of claims 1-5 with a metal acetate to form a crystalline porous metal-organic framework (MOF);
reacting a phosphorous ligand of any one of Formula II, Formula IV, Formula VI or Formula VIII of any one of claims 6-8 with the crystalline porous MOF in a reaction to form a heterogeneous catalyst precursor of Formula III, Formula V, Formula VII or Formula IX, respectively, of any one of claims 6-8; and
reacting the Group VIII transition metal catalyst precursor compound of any one of claims 9-12 with the heterogeneous catalyst precursor of any one of claims 6-8 to form the heterogeneous catalyst.

14. A method of producing an aldehyde, comprising:
providing a reaction mixture of an C3 to C12 olefin, synthesis gas and the heterogeneous catalyst of any one of claims 9-12; and
reacting the C3 to C12 olefin with synthesis gas in the presence of the heterogeneous catalyst in a hydroformylation process to produce the aldehyde.

15. The method of claim 14, further including separating the heterogeneous catalyst from the reaction mixture after producing the aldehyde wherein separating the heterogeneous catalyst includes:
purging synthesis gas from reaction mixture; and
drying the heterogeneous catalyst under vacuum to separate the heterogeneous catalyst from the reaction mixture.

## Patentansprüche

1. Kristallines poröses metallorganisches Gerüst (MOF), umfassend:
eine Vielzahl von nicht-katalytischen Metallionen; und
eine Vielzahl von Linkern, wobei die Vielzahl von nicht-katalytischen Metallionen mit der Vielzahl von Linkern koordiniert, um das kristalline poröse MOF zu bilden, wobei die Vielzahl von Linkern aus einer Verbindung von Formel I gebildet ist: 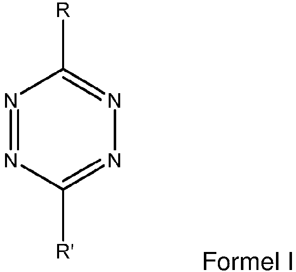
wobei R und R' jeweils unabhängig voneinander ausgewählt ist aus: 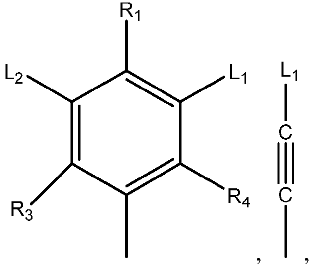 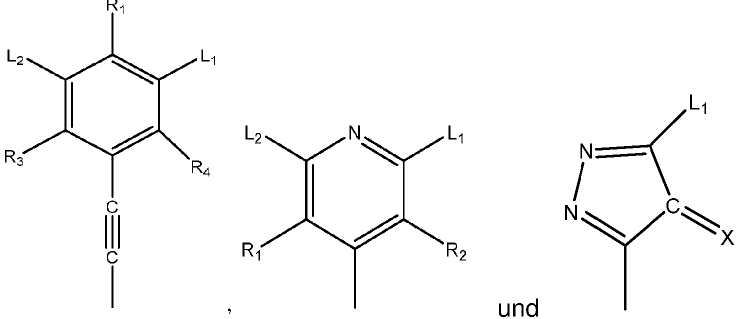
wobei R₁ und R₂ jeweils unabhängig voneinander H, C₁- bis C₃-Alkyl, -F, -Cl, -Br, -I oder -CF₃ sind;
wobei R₃ und R₄ jeweils unabhängig voneinander H oder C₁- bis C₃-Alkyl sind, wobei R₃ und R₄ eine Brückenstruktur bilden können;
X aus O und S ausgewählt ist;
L₁ und L₂ jeweils ausgewählt aus der Gruppe sind: 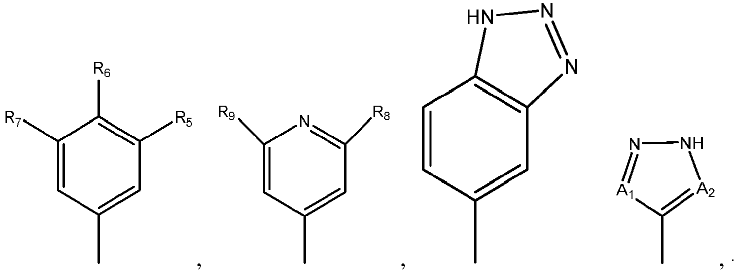 COOH, -OH und -NH₂,
wobei jedes von R₅, R₆ und R₇ unabhängig voneinander ausgewählt ist aus -H-OR₁₀, -COOR₁₁, -NR₁₂ -F, -Cl, -Br, -I oder --CF₃, wobei R₁₀, R₁₁ und R₁₂ jeweils unabhängig voneinander ausgewählt sind aus -H oder C₁- bis C₃-Alkyl;
wobei R₈ und R₉ jeweils unabhängig voneinander ausgewählt sind aus -H, -OH, -COOH oder -NH₂; und
A₁ und A₂ entweder C oder N sind; oder
wobei R und R' jeweils Folgendes ist: 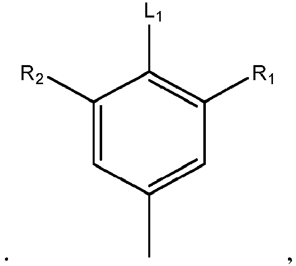 wobei L₁ ist 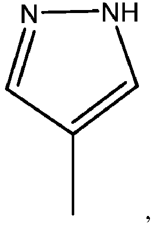 R₁ -H, -F oder -CF₃ ist und R₂ -H ist.

2. Kristallines poröses MOF nach Anspruch 1, wobei R und R' jeweils ist: 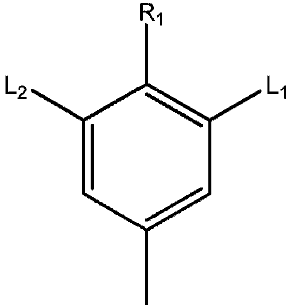 und L₁ und L₂ sind

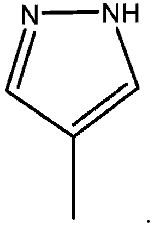

3. Kristallines poröses MOF nach Anspruch 2, wobei R₁ H ist.

4. Kristallines poröses MOF nach einem der Ansprüche 1 bis 3, wobei die Vielzahl von nicht-katalytischen Metallionen ausgewählt sind aus der Gruppe bestehend aus Nickel, Magnesium, Kupfer, Kobalt, Zirkonium, Eisen, Zink, Vanadium, Aluminium und Kombinationen davon.

5. Kristallines poröses MOF nach einem der Ansprüche 1 bis 3, wobei die Vielzahl von nicht-katalytischen Metallionen Nickel oder Magnesium sind.

6. Heterogener Katalysatorvorläufer, umfassend:
ein Reaktionsprodukt des kristallinen porösen MOF nach einem der Ansprüche 1 bis 5 und eines Phosphorliganden der Formel II: 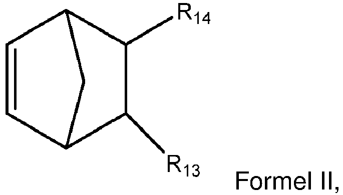
um den heterogenen Katalysatorvorläufer der Formel III zu bilden: 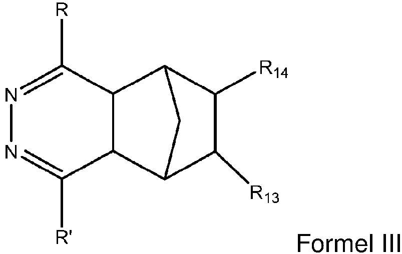 oder
einen Phosphorliganden der Formel IV: 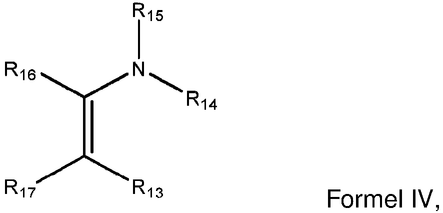
um den heterogenen Katalysatorvorläufer der Formel V zu bilden: 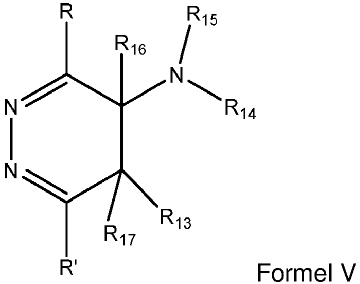 oder 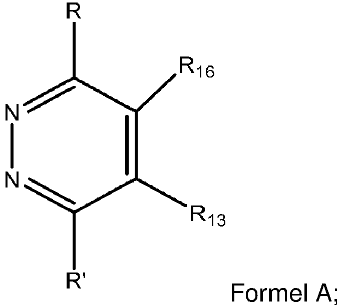 oder
einen Phosphorliganden der Formel VI: 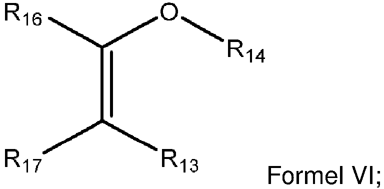
um den heterogenen Katalysatorvorläufer der Formel VII zu bilden: 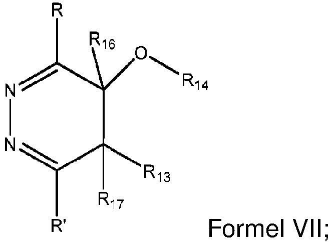 oder 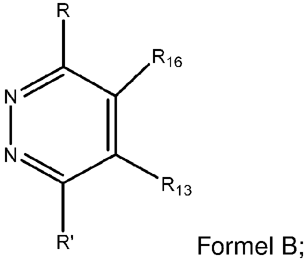
einen Phosphorliganden der Formel VIII: 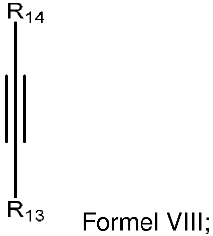 oder 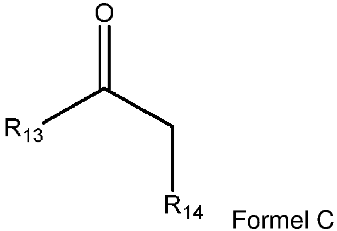 und einen Aminkatalysator,
um den heterogenen Katalysatorvorläufer der Formel IX zu bilden: 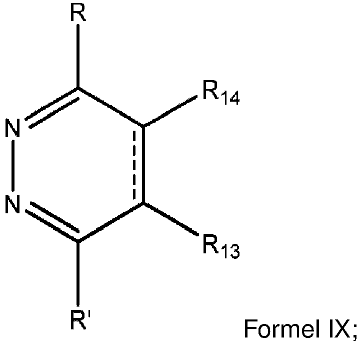 oder
einen Phosphorliganden der Formel D: 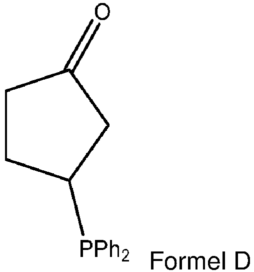
zur Bildung von: 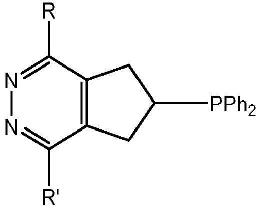
wobei R₁₆ und R₁₇ jeweils unabhängig voneinander -H oder C₁- bis C₃-Alkyl sind, und
wobei R₁₃ bis R₁₅ jeweils unabhängig voneinander ausgewählt sind aus -H, C₁- bis C₃₀-Alkyl und mindestens einem von: 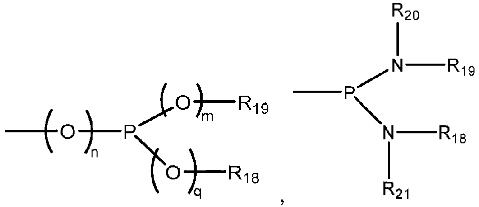 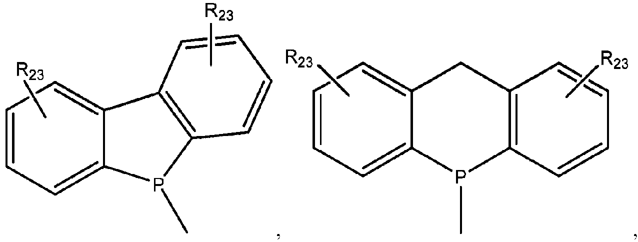 PPh₂ oder 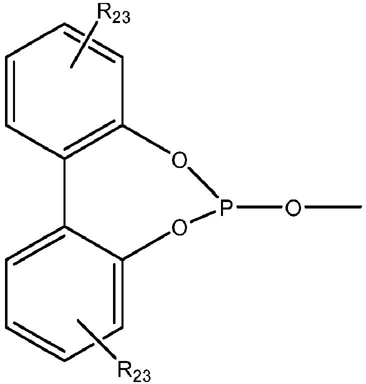
wobei n, m und q entweder 0 oder 1 sind, wobei (n,m,q) entweder (1,0,0), (1,1,0), (0,0,1), (0,1,1) oder (1,1,1) ist;
wobei R₁₈ bis R₂₁ jeweils ausgewählt sind aus C₅-C₁₀-Aryl, C₁-C₄-Alkyl oder -N(R₂₂)₂, wobei R₂₂ ausgewählt ist aus -H oder C₁- bis C₃-Alkyl; und
wobei R₂₃ jeweils ausgewählt ist aus C₅-C₁₀-Aryl, C₁-C₄-Alkyl, -OR₂₂ oder -N(R₂₂)₂, wobei R₂₂ ausgewählt ist aus -H oder C₁- bis C₃-Alkyl.

7. Heterogener Katalysatorvorläufer nach Anspruch 6, wobei der heterogene Katalysatorvorläufer die Formel III aufweist und R₁₃ und R₁₄ jeweils Diphenylphosphin sind.

8. Heterogener Katalysatorvorläufer nach Anspruch 6, wobei R₁₃ H ist und R₁₄ PPh₂ ist.

9. Heterogene Katalysatorzusammensetzung zur Hydroformylierung von Olefinen, umfassend:
ein Reaktionsprodukt des heterogenen Katalysatorvorläufers nach einem der Ansprüche 6 bis 8 mit einer Übergangsmetallkatalysatorvorläuferverbindung der Gruppe VIII.

10. Heterogene Katalysatorzusammensetzung nach Anspruch 9, wobei die Übergangsmetallkatalysatorvorläuferverbindung der Gruppe VIII Formel X aufweist:
M_{w}(L¹)ₓ(L²)_{y}(L³)_{z} Formel X
wobei das M aus der Gruppe ausgewählt ist, bestehend aus Rhodium (Rh), Kobalt (Co), Iridium (Ir), Ruthenium (Ru), Eisen (Fe), Nickel (Ni), Palladium (Pd), Platin (Pt) und Osmium (Os);
L¹, L² und L³ jeweils unabhängig voneinander ausgewählt sind aus der Gruppe, bestehend aus Wasserstoff, Carbonyl (CO), Cyclooctadien, Norbornen, Chlor, Sauerstoff, Bor, Fluorid, Bromid, Iodid, Nitrat, Acetat, Octanoat, 2-Ethylhexanoat, Triphenylphosphin (TPP) und Acetylacetonat (AcAc);
w eine ganze Zahl von 1 bis 6 ist; und
x, y und z jeweils unabhängig voneinander eine ganze Zahl von 0 bis 5 sind, wobei die Summe von x, y und z mindestens 1,0 beträgt.

11. Heterogener Katalysator nach Anspruch 10, wobei der Übergangsmetallkatalysator der Formel X aus der Gruppe ausgewählt ist, bestehend aus Rh(O₂C₅H₇)(CO)₂, Rh₂O₃, Rh₄(CO)₁₂, Rh₆(CO)₁₆, Rh(NO₃)₃, Bis(norbornadien)rhodium(I)-tetrafluoroborat und Bis(1,5-cyclooctadien)rhodium(I)-tetrafluoroborat.

12. Heterogener Katalysator nach Anspruch 9, wobei der heterogene Katalysatorvorläufer in einem molaren Überschuss, basierend auf dem Phosphorgehalt, relativ zu der Übergangsmetallkatalysatorvorläuferverbindung der Gruppe VIII bereitgestellt wird.

13. Verfahren zum Bilden eines heterogenen Katalysatorvorläufers, umfassend:
Umsetzen einer Verbindung der Formel I nach einem der Ansprüche 1 bis 5 mit einem Metallacetat zur Bildung eines kristallinen porösen metallorganischen Gerüsts (MOF);
Umsetzen eines Phosphorliganden nach einer der Formel II, Formel IV, Formel VI oder Formel VIII nach einem der Ansprüche 6 bis 8 mit dem kristallinen porösen MOF in einer Reaktion zur Bildung eines heterogenen Katalysatorvorläufers der Formel III, Formel V, Formel VII bzw. Formel IX nach einem der Ansprüche 6 bis 8; und
Umsetzen der Übergangsmetallkatalysatorvorläuferverbindung der Gruppe VIII nach einem der Ansprüche 9 bis 12 mit dem heterogenen Katalysatorvorläufer nach einem der Ansprüche 6 bis 8 zur Bildung des heterogenen Katalysators.

14. Verfahren zum Herstellen eines Aldehyds, umfassend:
Bereitstellen eines Reaktionsgemisches aus einem C3- bis C12-Olefin, Synthesegas und dem heterogenen Katalysator nach einem der Ansprüche 9 bis 12; und
Umsetzen des C3- bis C12-Olefins mit Synthesegas in Gegenwart des heterogenen Katalysators in einem Hydroformylierungsprozess zur Herstellung des Aldehyds.

15. Verfahren nach Anspruch 14, ferner einschließend das Abtrennen des heterogenen Katalysators aus dem Reaktionsgemisch nach der Herstellung des Aldehyds, wobei das Abtrennen des heterogenen Katalysators einschließt:
Spülen von Synthesegas aus dem Reaktionsgemisch; und
Trocknen des heterogenen Katalysators unter Vakuum, um den heterogenen Katalysator von dem Reaktionsgemisch abzutrennen.

## Revendications

1. Réseau organométallique (MOF) poreux cristallin comprenant :
une pluralité d'ions métalliques non catalytiques ; et
une pluralité de coupleurs, dans lequel la pluralité d'ions métalliques non catalytiques se coordonnent avec la pluralité de coupleurs pour former le MOF poreux cristallin, dans lequel la pluralité de coupleurs sont formés à partir d'un composé de Formule I : 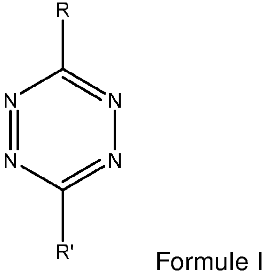
où chacun de R et de R' est indépendamment choisi parmi : 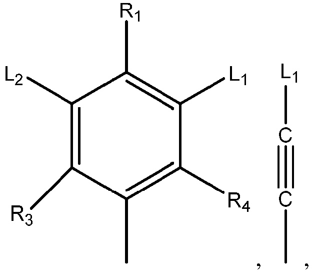 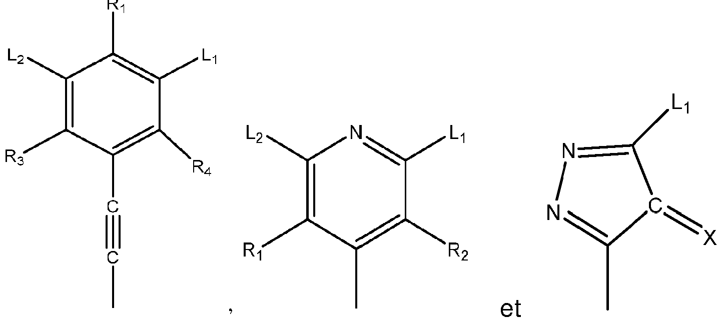
où chacun de R₁ et de R₂ est indépendamment H, alkyle en C₁ à C₃, -F,-Cl, -Br, -I, ou -CF₃ ;
où chacun de R₃ et R₄ est indépendamment H ou alkyle en C₁ à C₃, où R₃ et R₄ peuvent former une structure de pont ;
X est choisi parmi O et S ;
L₁ et L₂ sont chacun choisis dans le groupe : 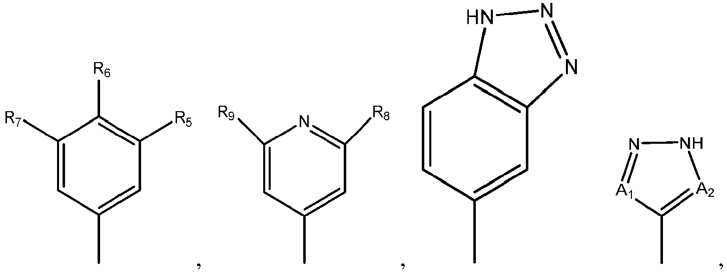 COOH, -OH et -NH₂
où chacun de R₅, R₆ et R₇ est choisi indépendamment parmi -H, -OR₁₀,-COOR₁₁, -NR₁₂, -F, -CI, -Br, -I, ou --CF₃, dans lequel R₁₀, R₁₁ et R₁₂ sont chacun choisi indépendamment parmi -H ou alkyle en C₁ à C₃ ;
où chacun de R₈ et R₉ est choisi indépendamment parmi -H, -OH,-COOH, ou -NH₂ ; et
A₁ et A₂ sont soit C, soit N ; ou
où chacun de R et de R' est : 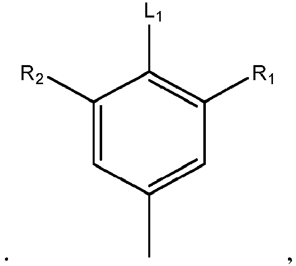 où L₁ est 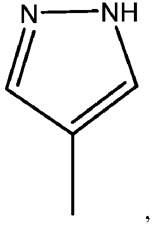 R₁ est -H, -F ou -CF₃ et R₂ est -H.

2. MOF poreux cristallin selon la revendication 1, dans lequel chacun de R et de R' est : 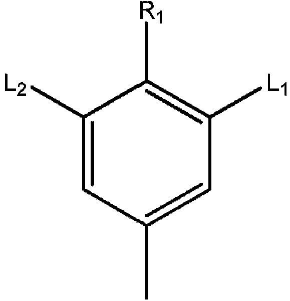 et L₁ et L₂ sont

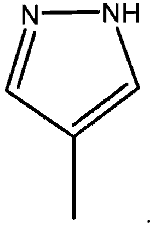

3. MOF poreux cristallin selon la revendication 2, dans lequel R₁ est H.

4. MOF poreux cristallin selon l'une quelconque des revendications 1 à 3, dans lequel la pluralité d'ions métalliques non catalytiques sont choisis dans le groupe constitué de nickel, magnésium, cuivre, cobalt, zirconium, fer, zinc, vanadium, aluminium et leurs combinaisons.

5. MOF poreux cristallin selon l'une quelconque des revendications 1 à 3, dans lequel la pluralité d'ions métalliques non catalytiques est du nickel ou du magnésium.

6. Précurseur de catalyseur hétérogène, comprenant :
un produit de réaction du MOF poreux cristallin selon l'une quelconque des revendications 1 à 5 et un ligand phosphoreux de Formule II : 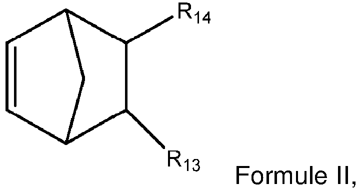
pour former le précurseur de catalyseur hétérogène de Formule III : 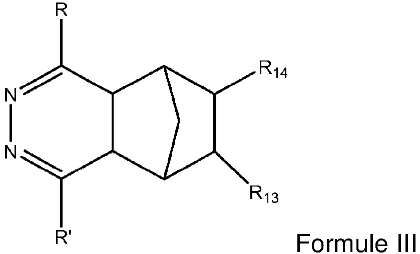 ou
un ligand phosphoreux de Formule IV : 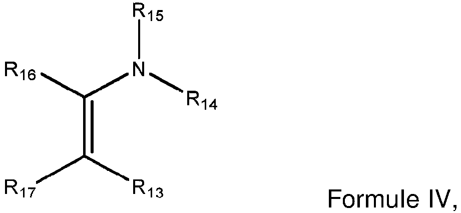
pour former le précurseur de catalyseur hétérogène de Formule V : 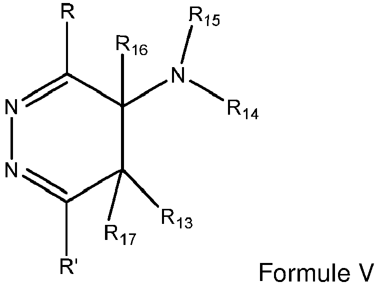 ou 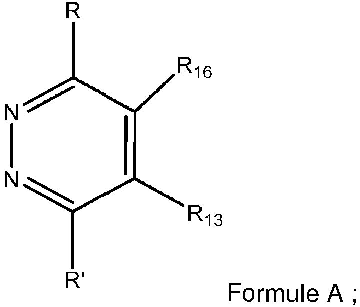 ou
un ligand phosphoreux de Formule VI : 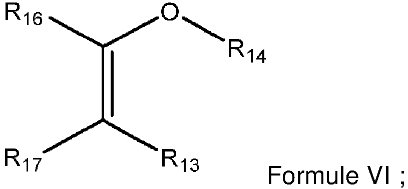
pour former le précurseur de cata 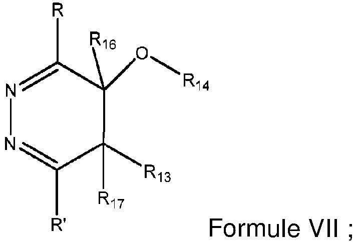 lyseur hétérogène de Formule VII : ou 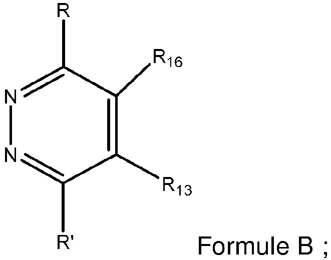
un ligand phosphoreux de Formule VIII : 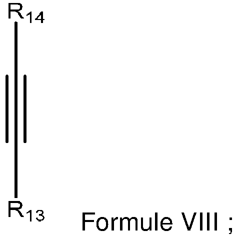 ou 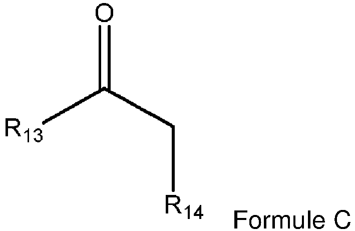 et un catalyseur amine
pour former le précurseur de catalyseur hétérogène de Formule IX : 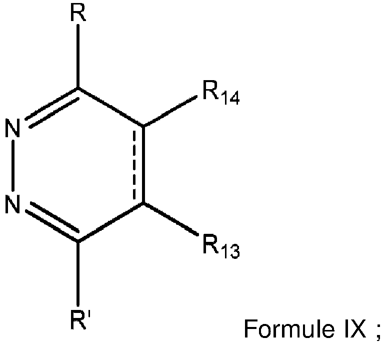 ou
un ligand phosphoreux de Formule D : 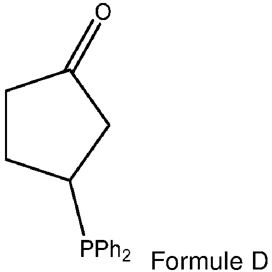
pour former : 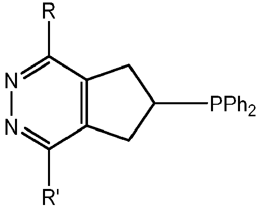
où R₁₆ et R₁₇ sont chacun indépendamment -H ou alkyle en C₁ à C₃ ; et
où R₁₃ à R₁₅ sont chacun choisi indépendamment parmi -H, alkyle en C₁ à C₃₀ et au moins l'un parmi : 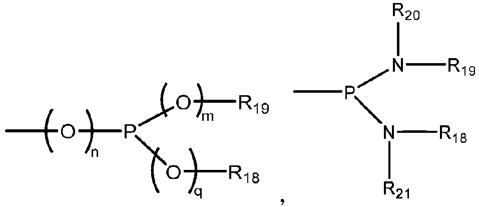 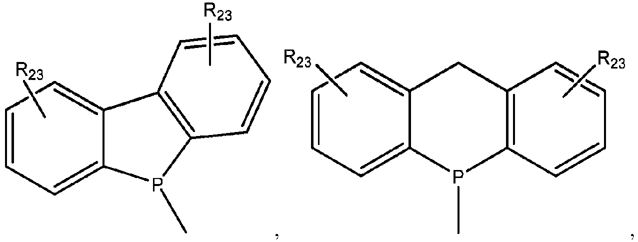 PPh₂ ou 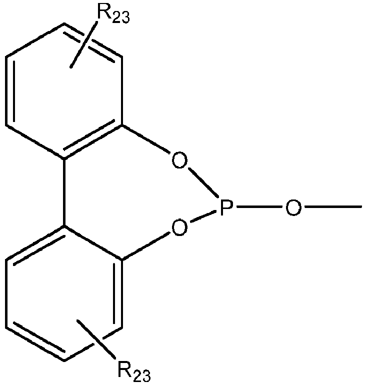
où n, m, et q sont soit 0 soit 1, où (n,m,q) est soit (1,0,0), (1,1,0), (0,0,1), (0,1,1) ou (1,1,1) ;
où chacun de R₁₈ à R₂₁ est choisi parmi aryle en C₅ à C₁₀, alkyle en C₁ à C₄ ou -N(R₂₂)₂, où R₂₂ est choisi parmi -H ou alkyle en C₁ à C₃ ; et
où chacun de R₂₃ est choisi parmi aryle en C₅ à C₁₀, alkyle en C₁ à C₄,-OR₂₂ ou -N(R₂₂)₂, où R₂₂ est choisi parmi -H ou alkyle en C₁ à C₃.

7. Précurseur de catalyseur hétérogène selon la revendication 6, dans lequel le précurseur de catalyseur hétérogène est de Formule III et R₁₃ et R₁₄ sont chacun diphénylphosphine.

8. Précurseur de catalyseur hétérogène selon la revendication 6, dans lequel R₁₃ est H et R₁₄ est PPh2.

9. Composition de catalyseur hétérogène pour l'hydroformylation d'oléfines, comprenant :
un produit de réaction du précurseur de catalyseur hétérogène selon l'une quelconque des revendications 6 à 8 avec un composé de précurseur de catalyseur de métal de transition du groupe VIII.

10. Composition de catalyseur hétérogène selon la revendication 9, dans laquelle le composé précurseur de catalyseur de métal de transition du groupe VIII est de formule X :
M_{w}(L¹)ₓ(L²)_{y}(L³)_{z} Formule X
où le M est choisi dans le groupe constitué de rhodium (Rh), cobalt (Co), iridium (Ir), ruthénium (Ru), fer (Fe), nickel (Ni), palladium (Pd), platine (Pt) et osmium (Os) ;
L¹, L² et L³ sont chacun choisis indépendamment dans le groupe constitué d'hydrogène, carbonyle (CO), cyclo-octadiène, norbornène, chlore, oxygène, bore, fluorure, bromure, iodure, nitrate, acétate, octanoate, 2-éthylhexanoate, triphénylphosphine (TPP) et acétylacétonate (AcAc) ;
w est un nombre entier allant de 1 à 6 ; et
x, y et z sont chacun indépendamment un nombre entier allant de 0 à 5, où la somme de x, y et z vaut au moins 1,0.

11. Catalyseur hétérogène selon la revendication 10, dans lequel le catalyseur de métal de transition de Formule X est choisi dans le groupe constitué de Rh(O₂C₅H₇)(CO)₂, Rh₂O₃, Rh₄(CO)₁₂, Rh₆(CO)₁₆, Rh(NO₃)₃, tétrafluoroborate de bis(norbornadiène)rhodium(I) et tétrafluoroborate de bis(1,5-cyclooctadiène)rhodium(I).

12. Catalyseur hétérogène selon la revendication 9, dans lequel le précurseur de catalyseur hétérogène est fourni dans un excès molaire, en fonction de la teneur en phosphore, par rapport au composé de précurseur de catalyseur de métal de transition du groupe VIII.

13. Procédé de formation d'un catalyseur hétérogène, comprenant :
le fait de faire réagir un composé de Formule I selon l'une quelconque des revendications 1 à 5 avec un acétate métallique pour former un réseau organométallique (MOF) poreux cristallin ;
le fait de faire réagir un ligand phosphoreux de l'une quelconque de la Formule II, de la Formule IV, de la Formule VI ou de la Formule VIII selon l'une quelconque des revendications 6 à 8 avec le MOF poreux cristallin dans une réaction pour former un précurseur de catalyseur hétérogène de Formule III, Formule V, Formule VII ou Formule IX, respectivement, selon l'une quelconque des revendications 6 à 8 ; et
le fait de faire réagir le composé précurseur de catalyseur de métal de transition du groupe VIII selon l'une quelconque des revendications 9 à 12 avec le précurseur de catalyseur hétérogène selon l'une quelconque des revendications 6 à 8 pour former le catalyseur hétérogène.

14. Procédé de production d'un aldéhyde comprenant :
la fourniture d'un mélange réactionnel d'une oléfine en C₃ à C₁₂, d'un gaz de synthèse et du catalyseur hétérogène selon l'une quelconque des revendications 9 à 12 ; et
le fait de faire réagir l'oléfine en C₃ à C₁₂ avec du gaz de synthèse en présence du catalyseur hétérogène dans un processus d'hydroformylation pour produire l'aldéhyde.

15. Procédé selon la revendication 14, comportant en outre la séparation du catalyseur hétérogène du mélange réactionnel après production de l'aldéhyde, dans lequel la séparation du catalyseur hétérogène comporte :
la purge du gaz de synthèse du mélange réactionnel ; et
le séchage sous vide du catalyseur hétérogène pour séparer le catalyseur hétérogène du mélange réactionnel.
